# EUROPEAN PATENT SPECIFICATION

(11) **EP 2 766 035 B1**
(45) Date of publication and mention of the grant of the patent: **28.03.2018**
(21) Application number: 12780684.2
(22) Date of filing: 10.10.2012
(51) Int. Cl.: A61K 9/00, A61K 38/20, A61K 39/395, A61K 45/06, A61P 35/00

(54) **COMBINATION MEDICAMENT COMPRISING IL-12 AND AN AGENT FOR BLOCKADE OF T-CELL INHIBITORY MOLECULES FOR TUMOUR THERAPY**
KOMBINATIONSMEDIKAMENT MIT IL-12 UND EINEM MITTEL ZUR BLOCKADE VON T-ZELLEN-HEMMER-MOLEKÜLEN ZUR TUMORTHERAPIE
COMBINAISON MÉDICAMENTEUSE COMPRENANT DE L'IL-12 ET UN AGENT DE BLOCAGE DE MOLÉCULES INHIBITRICES DES LYMPHOCYTES T POUR UNE THÉRAPIE ANTI-TUMORALE

(30) Priority: 11.10.2011 EP 11184644; 10.11.2011 EP 11188625; 19.09.2012 EP 12185108
(43) Date of publication of application: 20.08.2014
(73) Proprietor: Universität Zürich Prorektorat MNW, 8006 Zürich (CH)
(72) Inventor: BECHER, Burkhard., CH-8124 Maur (CH); VOM BERG, Johannes., 8037 Zürich (CH)
(74) Representative: Junghans, Claas
(86) International application number: PCT/EP2012/070088
(87) International publication number: WO 2013/053775

(56) References cited:
- P. L. TRIOZZI: "Phase I Study of the Intratumoral Administration of Recombinant Canarypox Viruses Expressing B7.1 and Interleukin 12 in Patients with Metastatic Melanoma", CLINICAL CANCER RESEARCH, vol. 11, no. 11, 1 June 2005 (2005-06-01), pages 4168-4175, XP055046157, ISSN: 1078-0432, DOI: 10.1158/1078-0432.CCR-04-2283
- SABEL ET AL: "Synergistic effect of intratumoral IL-12 and TNF-alpha microspheres: systemic anti-tumor immunity is mediated by both CD8+ CTL and NK cells", SURGERY, C.V. MOSBY CO., ST. LOUIS, US, vol. 142, no. 5, 2 November 2007 (2007-11-02), pages 749-760, XP022320882, ISSN: 0039-6060, DOI: 10.1016/J.SURG.2007.05.008
- ZHONG RUI-KUN ET AL: "Induction, selection and expansion of acute myeloid leukemia reactive autologous T cells for adoptive immunotherapy", BLOOD; 47TH ANNUAL MEETING OF THE AMERICAN-SOCIETY-OF-HEMATOLOGY; ATLANTA, GA, USA; DECEMBER 10 -13, 2005, AMERICAN SOCIETY OF HEMATOLOGY, US, vol. 106, no. 11 part 1, 16 November 2005 (2005-11-16), pages 1-2, XP009157602, ISSN: 0006-4971
- P. E. FECCI ET AL: "Systemic CTLA-4 Blockade Ameliorates Glioma-Induced Changes to the CD4+ T Cell Compartment without Affecting Regulatory T-Cell Function", CLINICAL CANCER RESEARCH, vol. 13, no. 7, 1 April 2007 (2007-04-01), pages 2158-2167, XP055022031, ISSN: 1078-0432, DOI: 10.1158/1078-0432.CCR-06-2070
- YUNHUI LIU ET AL: "In situ adenoviral interleukin 12 gene transfer confers potent and long-lasting cytotoxic immunity in glioma", CANCER GENE THERAPY, vol. 9, no. 1, 1 January 2002 (2002-01-01) , pages 9-15, XP055046148, ISSN: 0929-1903, DOI: 10.1038/sj.cgt.7700399

## Description

The present invention is as defined in the present claims relates to compositions and methods for treating cancer, in particular, to immunotherapy of malignant neoplastic disease such as glioma, by administering an effective dose of a polypeptide with IL-12 biological activity and a non-agonist ligand of a CTLA-4.

Glioblastoma multiforme (GBM) is the most malignant astrocytic tumour. GBM exhibits an invasive and destructive growth pattern; it is the most common and most aggressive malignant primary brain tumour in humans, accounting for 20 % of all intracranial tumours. In most European countries and North America, GBM incidence is in the range of 3-3.5 new cases per 100'000 population per year. The clinical history of the disease is usually short (less than 3 months in more than 50% of cases) and patients diagnosed with GBM show a median survival of 14-18 months despite aggressive surgery, radiation, and chemotherapy. The ability of gliomas to withstand conventional treatment regimens is one of the greatest challenges of modern neuro-oncology.

Interleukin (IL)-12 is the prototype of a group of heterodimeric cytokines with predominantly inflammatory properties. IL-12 polarizes naive helper T-cells to adopt a TH1 phenotype and stimulates cytotoxic T and NK-cells. IL-12 binds to the IL-12 receptor (IL-12R), which is a heterodimeric receptor formed by IL-12R-β1 and IL-12R-β2. The receptor complex is primarily expressed by T cells, but also other lymphocyte subpopulations have been found to be responsive to IL-12.

The therapeutic application of IL-12 in various tumour entities has been suggested. Clinical trials in cancer patients, however, had to be halted since systemic application evoked serious adverse events at effective doses, including fatalities. While research in recent years has mainly focused on various administration routes of IL-12, there remain open questions on the exact mechanisms by which IL-12 exerts its tumour-suppressive properties.

CTLA-4 and PD-1 are both members of the extended CD28/CTLA-4 family of T cell regulators. PD-1 is expressed on the surface of activated T cells, B cells and macrophages. PD-1(CD279; Uniprot Q15116) has two ligands, PD-L1 (B7-H1, CD274) and PD-L2 (B7-DC, CD273), which are members of the B7 family.

CTLA-4 (Uniprot ID No P16410) is expressed on the surface of T helper cells and transmits an inhibitory signal to T lymphocytes. CTLA-4 and CD28 bind to CD80 (B7-1) and CD86 (B7-2) on antigen-presenting cells. CTLA-4 transmits an inhibitory signal to T cells, whereas CD28 transmits a stimulatory signal. Systemic anti-CTLA-4 treatment has been approved for clinical use and demonstrates clinical benefit. It is being further tested for various other solid cancers (Hodi et al., N Engl J Med 363, 711-723 (2010); Graziani et al., Pharmacol Res (2012) Jan;65(1):9-22). A commercial antibody against CTLA-4 is available under the generic name ipilimumab (marketed as *Yervoy*).

The glycoprotein immunoglobulin G (IgG) is a major effector molecule of the humoral immune response in man. There are four distinct subgroups of human IgG designated IgG1, IgG2, IgG3 and IgG4. The four subclasses show more than 95% homology in the amino acid sequences of the constant domains of the heavy chains, but differ with respect to structure and flexibility of the hinge region, especially in the number of inter-heavy chain disulfide bonds in this domain. The structural differences between the IgG subclasses are also reflected in their susceptibility to proteolytic enzymes, such as papain, plasmin, trypsin and pepsin.

Only one isoform of human IgG4 is known. In contrast to human IgG1, IgG2 and IgG3, human IgG4 does not activate complement. Furthermore, IgG4 is less susceptible to proteolytic enzymes compared to IgG2 and IgG3.

The problem underlying the present invention is the provision of improved means and methods for treating solid cancer, in particular glioma.

In the course of a study focused on the clinical therapeutic potential of IL-12 in advanced-stage GBM in a relevant rodent model, it was surprisingly found that the combination of IL-12 with a blockade of co-inhibitory signals with anti-CTLA-4 antibody leads to almost complete tumour eradication and cure even at advanced disease stages.

According to a first aspect of the invention, a combination medicament is provided for use in the therapy of solid tumours, particular brain tumours, particularly glioma, which comprises
- an IL-12 polypeptide, or a nucleic acid expression vector comprising a sequence encoding said IL-12 polypeptide, and
- a non-agonist CTLA-4 ligand
wherein the IL-12 polypeptide, or the nucleic acid expression vector, is provided by administration into a tumour, into the vicinity of a tumour, or to the lymph node associated with a tumour.

In the context of the present invention, an IL-12 polypeptide is a polypeptide having an amino acid sequence comprising the sequence of p35 (Uniprot ID 29459, SEQ ID 05) or a functional homologue thereof, and comprising the sequence of p40 (Uniprot ID29460, SEQ ID 06) or a functional homologue thereof. In one embodiment, the IL-12 polypeptide has an amino acid sequence comprising both p35 and p40 sequences or homologues thereof as part of the same continuous amino acid chain. In another embodiment, the IL-12 polypeptide comprises two distinct amino acid chains, one comprising the p35 sequence and another one comprising the p40 sequence. The terminology "IL-12 polypeptide" does not preclude the presence of non-IL-12 sequences, for example immunoglobulin sequences and fragments thereof, fused to the IL-12 sequences described herein.

The IL-12 polypeptide has a biological activity of IL-12. A biological activity of IL-12 in the context of the present invention is the stimulation of NK or T cells by said IL-12 polypeptide, most prominently the stimulation of T effector cells acting through perforin.

In one embodiment of the combination medicament, said IL-12 polypeptide comprises a polypeptide sequence at least 95%, 96%, 97%, 98% or 99% identical to the sequence of human p35 (SEQ ID 05), and a polypeptide sequence at least 95%, 96%, 97%, 98% or 99% identical to the sequence of human p40 (SEQ ID 06).

Identity in the context of the present invention is a single quantitative parameter representing the result of a sequence comparison position by position. Methods of sequence comparison are known in the art; the BLAST algorithm available publicly is an example.

In one embodiment, said IL-12 polypeptide is a recombinant human IL-12. In one embodiment, said IL-12 polypeptide is a synthetic human IL-12. In one embodiment, said IL-12 polypeptide is a fusion peptide comprising the crystallisable fragment (Fc region) of a human immunoglobulin. According to one embodiment, the IL-12 polypeptide comprises a crystallisable fragment of human immunoglobulin G. A crystallizable fragment in the context of the present invention refers to the second and third constant domain of the IgG molecule. The fragment crystallizable region (Fc region) is the tail region of an immunoglobulin antibody that interacts with cell surface receptors (Fc receptors) and proteins of the complement system. In IgG antibody isotypes, the Fc region is composed of two identical protein fragments, derived from the second and third constant domains of the antibody's two heavy chains.

According to one embodiment, the IL-12 polypeptide comprises a crystallisable fragment of human immunoglobulin G4. According to one embodiment, the IL-12 polypeptide has or comprises the sequence of SEQ ID 01. According to another embodiment, the IL-12 polypeptide comprises a sequence at least 95%, 96%, 97%, 98% or 99% identical to the sequence of SEQ ID 01.

Embodiments wherein IL-12 polypeptide chains are fused to immunoglobulin Fc fragments show different pharmacokinetic behaviour in comparison to the recombinant cytokine, which for some applications may confer a benefit.

In one embodiment, the IL-12 polypeptide component of the combination medicament is provided as a dosage form for local (intratumoural) administration or delivery. Such dosage form for local (intratumoural) administration may be a slow-release form or depot form, from which said IL-12 polypeptide is released over a number of hours to weeks. In one embodiment, the IL-12 polypeptide component of the combination medicament is administered via convection enhanced delivery (CED) or a variation thereof, for example the device shown in US2011137289 (A1).

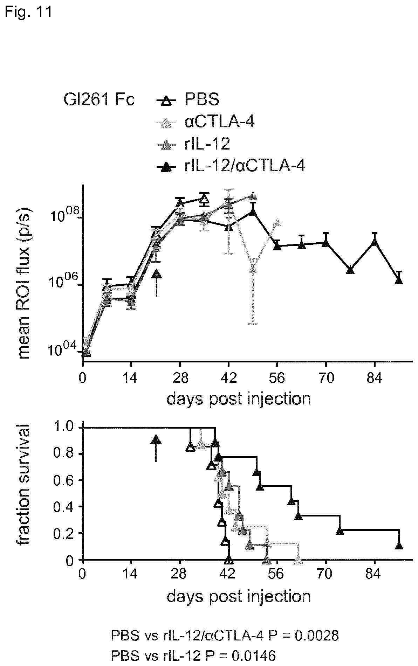

In one embodiment, the IL-12 polypeptide is administered systemically together with systemic CTLA-4 blockade. Heterodimeric recombinant IL-12 (peprotech) applied systemically together with systemic CTLA-4 blockade (i.p.) achieved a significant improvement in survival in comparison to either agent administered by itself (see Fig. 11).

In the context of the present invention, a non-agonist CTLA-4 ligand is a molecule that binds selectively to CTLA-4 under conditions prevailing in peripheral blood, without triggering the biological effect of CTLA-4 interaction with any of the physiological ligands of CTLA-4, particularly CD80 and/or CD86.

A non-agonist CTLA-4 ligand in the sense of the invention refers to a molecule that is capable of binding to CTLA-4 with a dissociation constant of at least 10-7 M⁻¹, 10⁻⁸ M⁻¹ or 10⁻⁹ M⁻¹ and which inhibits the biological activity of its respective target.

A non-agonist polypeptide ligand may be an antibody, an antibody fragment, an antibody-like molecule or an oligopeptide, any of which binds to and thereby inhibits CTLA-4.

An antibody fragment may be a Fab domain or an Fv domain of an antibody, or a single-chain antibody fragment, which is a fusion protein consisting of the variable regions of light and heavy chains of an antibody connected by a peptide linker. The inhibitor may also be a single domain antibody, consisting of an isolated variable domain from a heavy or light chain. Additionally, an antibody may also be a heavy-chain antibody consisting of only heavy chains such as antibodies found in camelids. An antibody-like molecule may be a repeat protein, such as a designed ankyrin repeat protein (Molecular Partners, Zurich).

An oligopeptide according to the above aspect of the invention may be a peptide derived from the recognition site of a physiological ligand of CTLA-4. Such oligopeptide ligand competes with the physiological ligand for binding to CTLA-4.

Particularly, a non-agonist CTLA-4 ligand does not lead to attenuated T cell activity when binding to CTLA-4 on the surface on a T-cell. In certain embodiments, the term "non-agonist CTLA-4 ligand" " covers both antagonists of CTLA-4 and ligands that are neutral vis-à-vis CTLA-4 signalling. In some embodiments, non-agonist CTLA-4 ligands used in the present invention are able, when bound to CTLA-4, to sterically block interaction of CTLA-4 with its binding partners CD80 and/or CD86.

In one embodiment, said non-agonist CTLA-4 ligand is a gamma immunoglobulin binding to CTLA-4, without triggering the physiological response of CTLA-4 interaction with its binding partners CD80 and/or CD86.

Non-limiting examples for a CTLA-4 ligand are the clinically approved antibodies tremelimumab (CAS 745013-59-6) and ipilimumab (CAS No. 477202-00-9; Yervoy).

The term "gamma immunoglobulin" in this context is intended to encompass both complete immunoglobulin molecules and functional fragments thereof, wherein the function is binding to CTLA-4, PD-1 or PD-L1 (PD-L2) as laid out above.

The combination therapy comprises two distinct dosage forms, wherein said IL-12 polypeptide is provided as a dosage form for intratumoural delivery or local delivery in the vicinity of the tumour, and said non-agonist CTLA-4 ligand is provided as a dosage form for systemic delivery, particularly by intravenous injection. However, said non-agonist CTLA-4 ligand may also be locally applied in the same way as the IL-12 polypeptide. According to another embodiment, the IL-12 polypeptide is applied directly to the tumour draining lymph node.

According to another embodiment, the combination therapy comprises a dosage form whereby said IL-12 polypeptide is provided for intracranial delivery, e.g. by injection.

According to another aspect of the invention, a combination medicament is provided as set forth above, for use in a method of therapy of a malignant neoplastic disease, particularly solid cancerous lesions. In one embodiment, the malignant neoplastic disease is glioma. In one embodiment, the malignant neoplastic disease is a secondary brain tumour (brain metastasis of a neoplastic lesion arising outside the brain). In one embodiment, the disease is glioblastoma multiforme. In one embodiment, the malignant neoplastic disease is meningioma. In one embodiment, the malignant neoplastic disease is melanoma. In one embodiment, the malignant neoplastic disease is pancreatic cancer. In one embodiment, the malignant neoplastic disease is lung cancer. In one embodiment, the malignant neoplastic disease is prostate cancer. In one embodiment, the malignant neoplastic disease is bladder cancer.

Cancerous lesions have the propensity to spread into neighbouring tissue as well as distinct locations in the body, depending on their origin. 20-40% of all cancers develop brain metastasis; among those lung, breast and skin (melanoma) cancer are the most common sources of brain metastases (Sofietti et al., J Neurol 249, 1357-1369 (2002)). Similar to primary malignant brain tumours, brain metastases have a poor prognosis despite treatment and are quickly fatal. T-cells are the crucial effector cell population for IL-12 mediated tumor rejection in the brain. IL-12 together with anti-CTLA-4, anti-PD-1, anti-PD-L1 or anti-PD-L2 combination treatment addresses especially the T-cells to activate and repolarize them. Since brain metastases grow in the same immune-compartment as pirmary brain tumors, patients suffering from secondary brain tumors also benefit from the combination treatment.

In one embodiment, the combination medicament comprises an IL-12 polypeptide having a biological activity of IL-12 provided as a fusion protein comprising the amino acid of human p40, the amino acid sequence of human p35 and the crystallisable fragment of human IgG4, said IL-12 polypeptide being formulated as a dosage form for intratumoural delivery. According to this embodiment, the combination medicament further comprises an immunoglobulin G raised against CTLA-4 as a non-agonist CTLA-4 ligand formulated as a dosage form for systemic delivery. According to this embodiment, the combination medicament is provided for the treatment of malignant neoplastic disease, particularly for glioma, glioblastoma multiforme, meningioma, melanoma, pancreatic cancer, lung cancer, prostate cancer or bladder cancer.

According to an alternative aspect of the invention, a combination therapy comprises an IL-12 nucleic acid expression vector encoding an encoded IL-12 polypeptide having a biological activity of IL-12, and a a non-agonist CTLA-4 ligand.

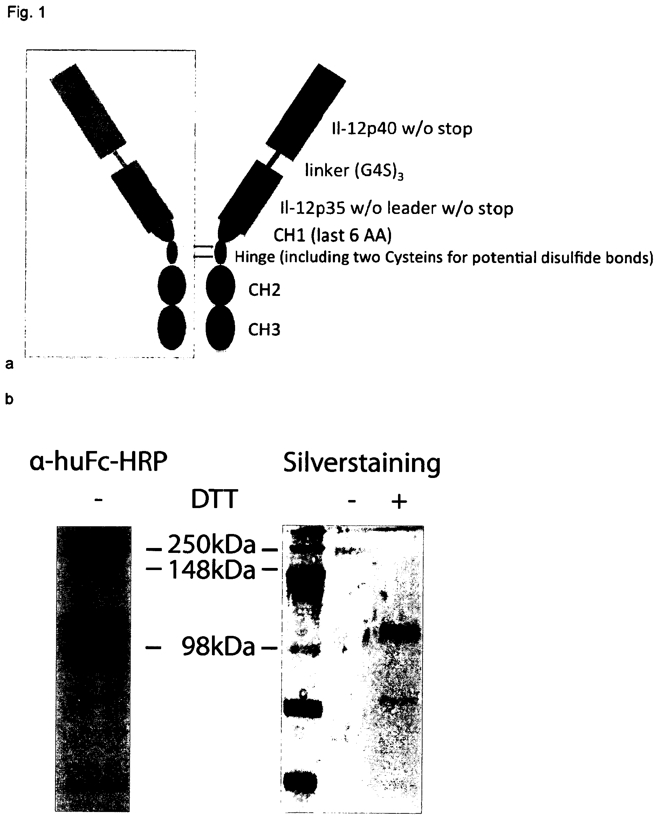

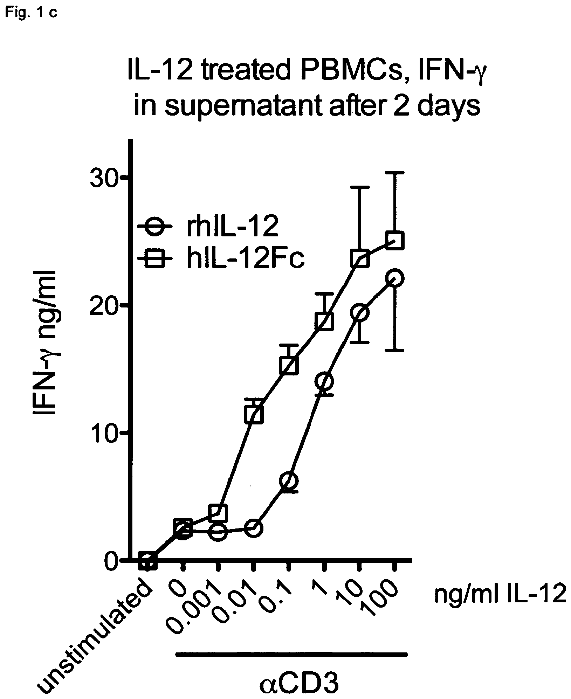

The CTLA-4 ligand may be embodied by polypeptides, particularly by antibodies, as set forth above. One non-limiting example for an encoded IL-12 polypeptide is a crystallisable immunoglobulin G fragment fused to the IL-12 constituent polypeptide chains, human IL-12 or a functional equivalent thereof. One non-limiting example is a fusion construct having the constituent polypeptides of IL-12 linked by a short amino acid sequence as depicted in Fig. 1, the amino acid sequence for which is given as SEQ ID 01 and the encoding nucleic acid sequence is given as SEQ ID 07.

The advantage of using fusion proteins cytokines and the crystallisable fragment of immunoglobulins rather than the recombinant cytokine is improved pharmacokinetics (Belladonna et al. J Immunol 168, 5448-5454 (2002); Schmidt, Curr Opin Drug Discov Devel 12, 284-295 (2009); Eisenring et al., Nat Immunol 11, 1030-1038 (2010)).

The IL-12 nucleic acid expression vector according to this aspect of the invention may, by way of non-limiting example, be a "naked" DNA expression plasmid comprising a nucleic acid sequence encoding the IL-12 polypeptide under control of a promoter sequence operable in a human tumour cell, for delivery into the tumour, for example by intracranial injection. The IL-12 nucleic acid expression vector may similarly be a viral vector, for example an adeno-associated virus, an adenovirus, a lentivirus or a herpes virus.

Such IL-12 nucleic acid expression vector may be provided as a dosage form for intratumoural delivery in combination with a protein non-agonist CTLA-4 ligand and/or a non-agonist PD-1 ligand as set forth above. Similarly, the scope of the present invention encompasses the use of such IL-12 nucleic acid expression vector, in combination with a non-agonist CTLA-4 ligand, in a method of making a combination medicament for use in therapy of malignant neoplastic disease, particularly glioma, glioblastoma multiforme, meningioma, melanoma, pancreatic cancer, lung cancer, prostate cancer or bladder cancer. Likewise, a method is provided for treating a patient suffering from malignant neoplastic disease, particularly glioma or other solid tissue tumours, comprising the administration of an IL-12 nucleic acid expression vector having a biological activity of IL-12, and a non-agonist CTLA-4 ligand and/or a non-agonist PD-1 ligand to said patient.

### Brief description of the Figures

- Fig. 1a: shows the structure and sequence of the fusion protein given in SEQ ID 01. The subunits p40 and p35 of IL-12 are depicted as rectangles. These subunits are connected by a linker (G₄S)₃. The subunits CH2, CH3 and the last six amino acids of CH1 of the crystallizable fragment of the immunoglobulin are shown as oblong circles.
- Fig. 1b: shows the fusion protein given SEQ ID 01, left picture shows an immunoblot using reducing conditions, developed with an HRP-coupled polyclonal anti-human Fc antibody, right picture shows a silver staining of the fusion protein under non-reducing (DTT -) and reducing conditions (DTT +)
- Fig. 1c: shows IFN-γ production in human peripheral blood monocytic cells (PBMCs) as assessed by enzyme linked immunosorbent assay (ELISA). Cells were stimulated either with commercially available heterodimeric recombinant human IL-12 (rhIL-12) or with the purified fusion protein given SEQ ID 01 (hIL-12Fc) in the presence of an antibody directed against CD3 (polyclonal T-cell stimulation). Unstimulated: neither IL-12 stimulation nor anti-CD3 stimulation (baseline control). Experiment was performed in triplicates, error bars denote s.e.m. data representative of three independent experiments

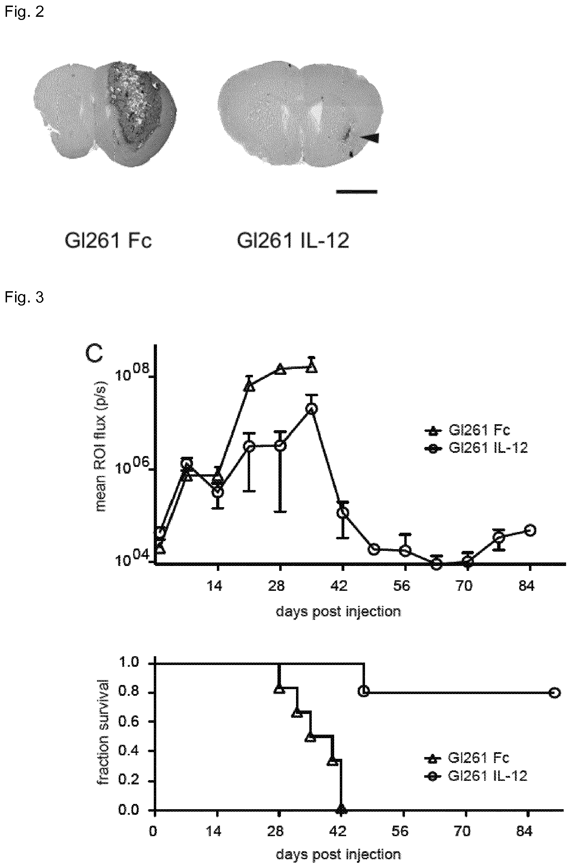

- Fig. 2: shows immunohistochemistry of formalin fixed tumour sections obtained from syngeneic C57/BI6 mice 5 weeks after challenge with 2x10⁴ GI261 IL-12Fc or GI261 Fc cells and stained with antibody against F4/80, counterstain hematoxylin (representative examples, n=6 mice per group). Scale bar indicates 2 mm, arrowhead indicates residual GI261 IL-12Fc (SEQ ID 02) tumour.
- Fig. 3: shows non-invasive Bioluminescence imaging (BLI) of mouse glioma in syngeneic C57/BI6 mice (n = 5-6 mice per group) after implantation of 2x10⁴ GI261 cells constitutively expressing *photinuspyralis* luciferase and releasing a fusion protein of IL-12 and the crystallizable fragment of mouse immunoglobulin G3 (GI261 IL-12Fc, (SEQ ID 02)) or Fc alone as control (GI261 Fc). Upper panel: Quantification of tumour growth which correlates to photon flux (p/s) in the region of interest (ROI) versus the days post injection of the modified glioma cells. Lower panel: Kaplan-Meier survival analysis. Data are representative of 2 independent experiments.

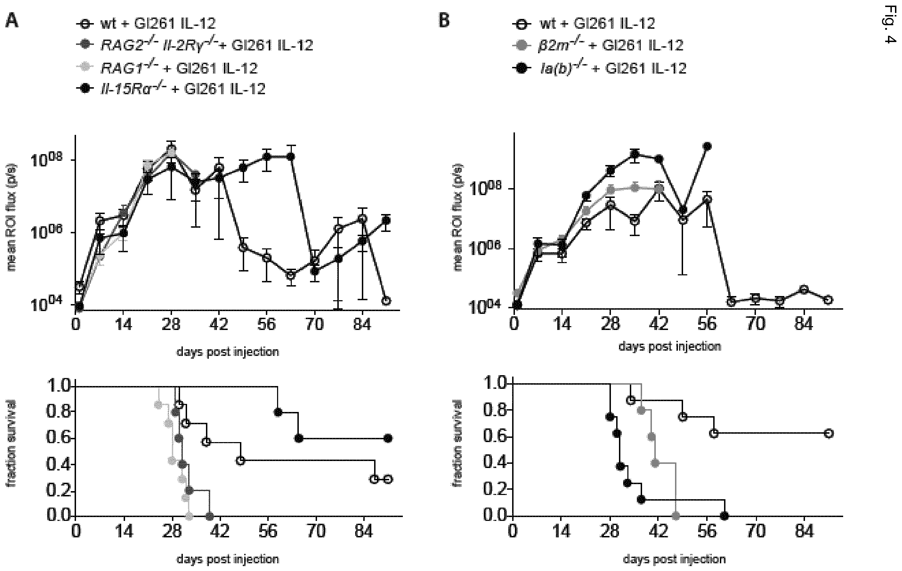

- Fig. 4: shows non-invasive Bioluminescence (BLI) imaging of mouse glioma in WT animals and different mouse mutants (n = 5-7mice per group) after implantation of 2x10⁴ GI261 cells constitutively expressing *photinuspyralis* luciferase and releasing a fusion protein of IL-12 and the crystallizable fragment of mouse immunoglobulin G3 (GI261 IL-12Fc, (SEQ ID 02)). Upper panel: Quantification of tumour growth via BLI imaging versus the days post injection of the modified glioma cells. Lower panel: Kaplan-Meier survival analysis. A) GI261 IL-12Fc were implanted in mice lacking T and B cells (*Rag1*^{*-*/*-*}) or NK cells (*II-15ra*^{*-*/*-*}) or lacking both T-, B-, NK cells and lymphoid tissue inducer like cells (*Rag2*^{*-*/}*⁻ II2rg*^{*-*/*-*}). B) GI261 IL-12Fc were implanted in mice deficient for MHCII (*Ia*(*b*)^{*-*/*-*}) and MHCI (*β2m*^{*-*/*-*}).(n=5-8 mice/group), lacking CD4 or CD8 positive T-cells, respectively. Data are representative of 2 independent experiments.

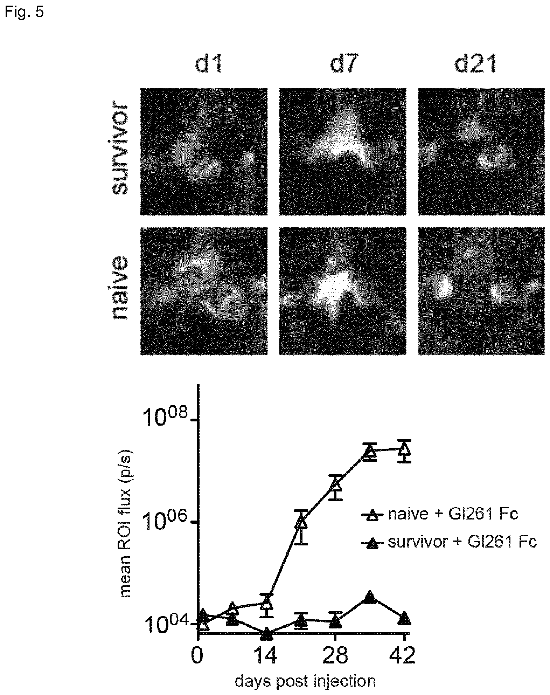

- Fig. 5: shows T cell memory formation in surviving wt animals that had been previously challenged with 2x10⁴ GI261 IL-12Fc (SEQ ID 02) cells. Examples for bioluminescence emitted from the brains of surviving wt animals that had been rechallenged with GI261 Fc cells compared to naïve wt animals is shown (upper panel, days 1,7 and 21 post rechallenge shown). Furthermore, bioluminescence of the tumours is shown in photons per second (p/s) in the region of interest (ROI) versus the days post injection of the modified glioma cells (lower panel). A rapid rejection of the control tumours in surviving wt animals was observed. While the measured luminescence at day 1 suggested identical seeding across the two groups, only the naïve mice exhibited a measurable signal at day 7 onwards, suggesting a rapid and effectively clearing anti-glioma memory response now independent of ectopically expressed pro-inflammatory cytokines (namely IL-12Fc, (SEQ ID 02)).(n=4-6 mice/group). Data are representative of 2 independent experiments.

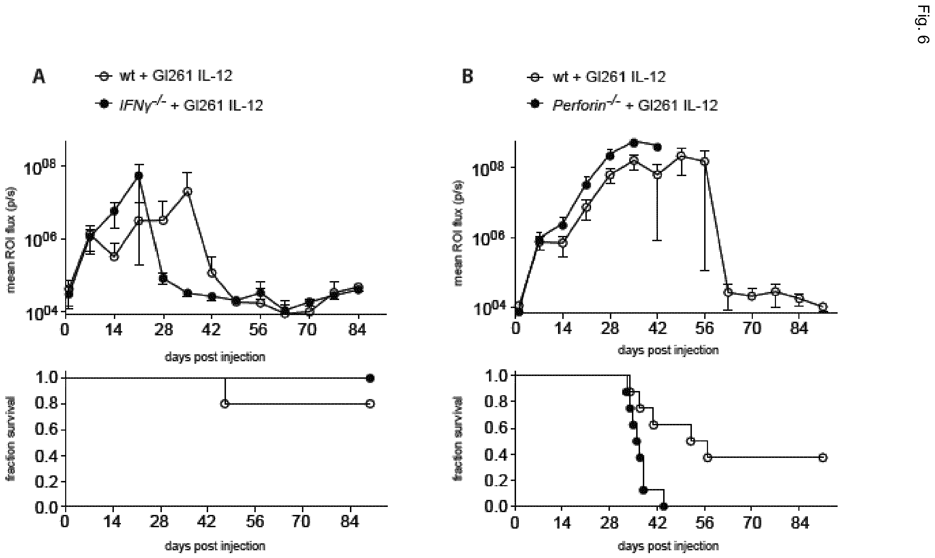

- Fig. 6: shows non-invasive Bioluminescence (BLI) imaging of mouse glioma in different mouse mutants (n = 4-8 mice per group) after implantation of 2x10⁴ GI261 Fccells constitutively expressing *photinuspyralis* luciferase and releasing a fusion protein of IL-12 and the crystallizable fragment of mouse immunoglobulin G3 (GI261 IL-12Fc (SEQ ID 02)). A) wt (open circles) and *IFNγ*^{*-*/*-*} (black circles) animals B) wt (open circles) and *Perforin*^{*-*/*-*} (black circles) animals. Quantification of tumour growth which correlates to photon flux (p/s) in the region of interest (ROI) versus the days post injection of the modified glioma cells are shown (upper panel). Lower panel: Kaplan-Meier survival analysis. Data are representative of 2 independent experiments.

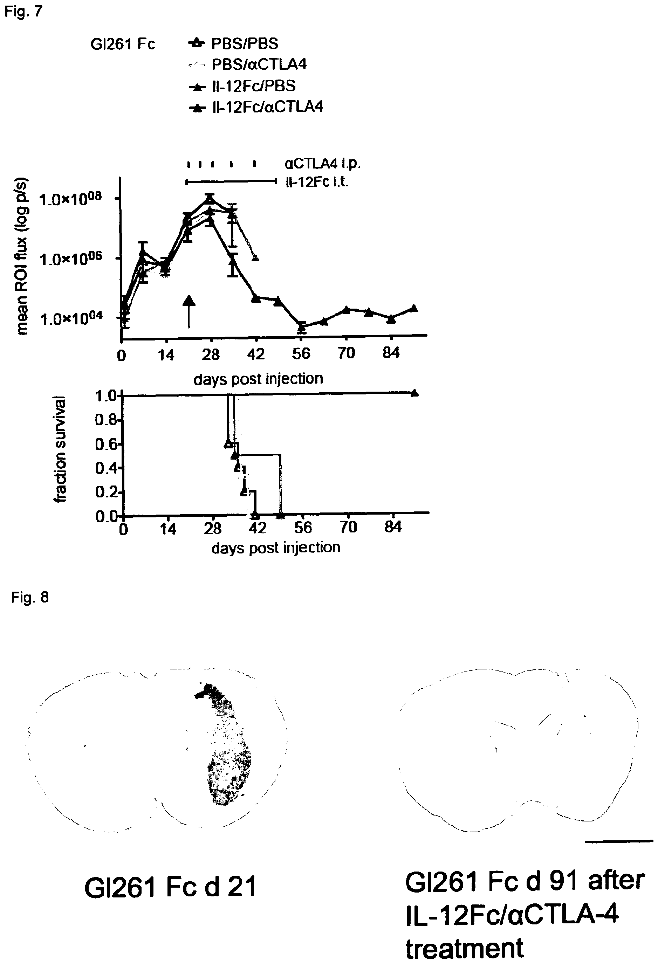

- Fig. 7: shows tumour growth in wt mice inoculated with 2x10⁴ GI261 Fc cells. Treatment started at day 21 (arrows). Osmotic minipumps delivering IL-12Fc (SEQ ID 02) (or PBS) into the tumour were implanted into glioma bearing animals. Animals received i.p. injections of αCTLA-4 blocking antibodies or PBS starting at day 22, followed by injections as indicated in figure. Upper graph: quantification of ROI photon flux of tumour bearing wt animals receiving the indicated treatment. Lower graph: Kaplan-Meier survival analysis of the animals above; PBS/PBS vs IL-12Fc/αCTLA-4 p=0.0045, PBS/PBS vs IL-12Fc/PBS p=0.3435, PBS/ αCTLA4 vs IL-12Fc/αCTLA-4 p=0.0101; Log-rank (Mantel-Cox) Test. Data representative of three independent experiments with 2-5 animals per group
- Fig. 8: shows immunohistochemistry of tumour sections obtained from syngenic C57/BI6 mice after challenge with GI261 Fc cells at day 21 and after local administration of IL-12Fc (SEQ ID 02) in combination with systemic CTLA-4 blockade as described in Example 5. The sections were stained with Hematoxylin and Eosin. Scale bar indicates 2 mm.

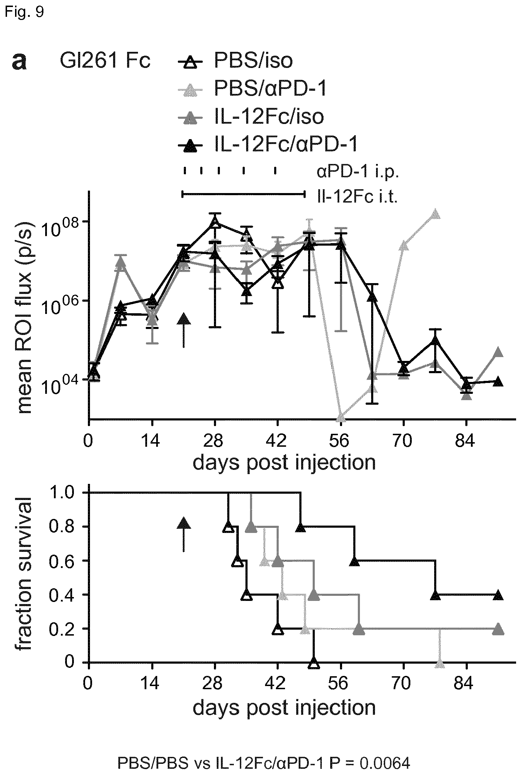

- Fig. 9: shows tumour growth in wt mice inoculated with 2x10⁴ GI261 Fc cells. Treatment started at day 21 (arrows). Osmotic minipumps delivering IL-12Fc (SEQ ID 02) into the tumour were implanted into glioma bearing animals. Animals received i.p. injections of αPD-1 blocking antibodies or isotype control antibodies starting at day 22, followed by injections as indicated in figure. Upper graph: quantification of ROI photon flux of tumour bearing wt animals receiving the indicated treatment. Lower graph: Kaplan-Meier survival analysis of the animals above; PBS/isotype vs IL-12Fc/αPD-1 p=0.0064, Log-rank (Mantel-Cox) Test. Data representative of one experiment with 5-6 animals per group.

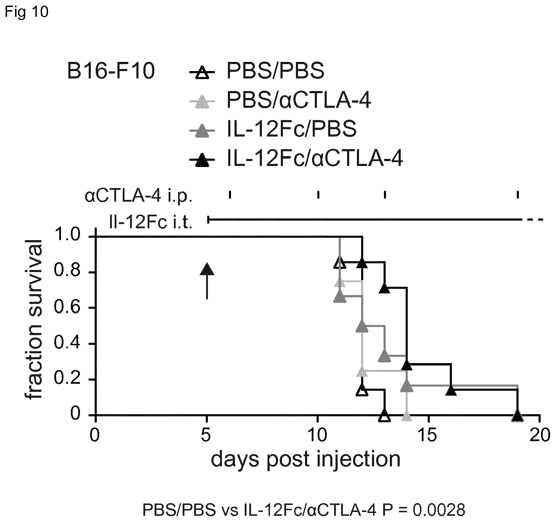

- Fig. 10: shows tumour growth in wt mice in inoculated with 50 B16-F10 cells. Treatment started at day 5 (arrow). Osmotic minipumps delivering IL-12Fc (SEQ ID 02) into the tumour were implanted into glioma bearing animals. Animals received i.p. injections of αCTLA-4 blocking antibodies or PBS starting at day 6, followed by injections as indicated in figure. Kaplan-Meier survival analysis PBS/PBS vs IL-12Fc/αCTLA-4 p=0.0028, Log-rank (Mantel-Cox) Test. Data representative of one experiment with 6 animals per group.
- Fig. 11: shows systemic administration of recombinant heterodimeric IL-12 in combination with CTLA4 blockade 2x10⁴ GI261 Fc cells were injected into the right striatum of wt mice and tumor growth was followed for 90 days. Systemic treatment: at day 21 (arrow), tumor bearing animals were treated initially with 20µg αCTLA-4 mouse IgG2b (9D9) (filled light grey triangles, n=8), 200ng of recombinant heterodimeric IL-12 (rIL-12) (filled dark grey triangles, n=9) or a combination of both (filled black triangles, n=9) injected intraperitoneal (i.p.). The control group received phospate buffered saline (PBS) (filled open triangles, n=7). Treatment was sustained with 100µg αCTLA-4 or 100ng rIL-12 or a combination of both 3 times / week until the end of the experiment. Upper graph: quantification of ROI photon flux of tumor bearing wt animals receiving the indicated treatment. Lower graph: Kaplan-Meier survival analysis of the animals above; Log-rank (Mantel-Cox) Test was used to calculate the p-values indicated; Pooled data from two independent experiments.

### Examples

### Methods

### Animals

C57BL/6 mice were obtained from Janvier; *b2m*^{*-*/*-*}, *Ia(b)*^{*-*/}*⁻, II2rb2*^{*-*/}*⁻, Rag1*^{*-*/}*⁻, Rag2*^{*-*/}*⁻II2rg*^{*-*/}*⁻, Prf1*^{*-*/*-*} and *Ifng*^{*-*/*-*}mice were obtained from Jackson Laboratories. *II5ra*^{-/-}mice were provided by S. Bulfone-Paus. All animals were kept in house under specific pathogen-free conditions at a 12hour light/dark cycle with food and water provided *ad libitum.* All animal experiments were approved by the Swiss Cantonary veterinary office (16/2009).

### Mouse tumour cell lines

C57/BI6 murine glioma (GI261) cells (kindly provided by A. Fontana, Experimental Immunology, University of Zurich) were transfected with pGI3-ctrl (Promega) and pGK-Puro (kindly provided by T. Buch, Technical University Munich). Linearized constructs were electroporated in a 10:1 ratio using an eppendorf multiporator, then selected with 0.8µg/ml puromycin (Sigma-Aldrich) to generate luciferase-stable GI261 cells. A single clone was isolated by limiting dilution and passaged in vivo by intracranial tumour inoculation, followed by tumour dissociation after 4 weeks and re-selection in 0.8µg/ml puromycin. Subsequently, cells were electroporated with pCEP4-mIgG3, pCEP4-mll-12mlgG3 (SEQ ID 09) and pCEP4-mII-23mIgG3 (SEQ ID 08) (Eisenring et al, 2010) and bulk-selected with 0.8µg/mlpuromycin and 0.23mg/ml hygromycin (Sigma-Aldrich). Cytokine production was detetected by ELISA (OptEIA II-12/23p40, BD Pharmingen) and rt-PCR (IgG3fw: ACACACAGCCTGGACGC (SEQ ID 03) IgG3rev: CATTTGAACTCCTTGCCCCT (SEQ ID 04)). GI261 cells and derived cell lines were maintained in Dulbecco's modified Eagle's medium (Gibco, Invitrogen) supplemented with 10% fetal calf serum (FCS) in presence of selection antibiotics as indicated above at 37°C and 10% CO₂. B16-F10 murine melanoma cells were purchased from ATCC.

### Expression and purification of IL-12Fc

IL-12Fc (SEQ ID 02) was expressed in 293T cells after calcium phosphate-mediated transfection according to standard protocols with 45µg of vector DNA (pCEP4-mIL-12IgG3, SEQ ID 09)/15cm tissue culture plate. Supernatant was harvested 3 days and 6 days after transfection, sterile filtered and diluted 1:1 in PBS. The protein was purified using a purifier (ÄktaPrime) over a protein G column (1 ml, HiTrap, GE Healthcare) eluted with 0.1 M glycine pH 2 and dialyzed over night in PBS pH 7.4. Concentration and purity of IL-12Fc (SEQ ID 02) was measured by ELISA (OptEIA II-12/23p40, BD Pharmingen) and SDS-PAGE followed by silverstaining and immunoblotting. IL-12Fc was detected with a rat anti mouse IL-12p40 antibody (C17.8, BioExpress) and a goat anti-rat HRP coupled antibody (Jackson). The same procedure was used for the expression of human IL-12Fc (SEQ ID 01, 07).

### Characterization of human IL-12Fc

Concentration and purity of human IL-12Fc (SEQ ID 01) was measured by ELISA (Human IL-12 (p70), Mabtech, #2455-1H-6) and SDS-PAGE followed by silver staining and immunoblotting. The human IgG4 tag was detected with an HRP-coupled goat anti human IgG antibody (#A0170, Sigma). For functional characterization of human IL-12Fc (SEQ ID 01) PBMCs, acquired according to the ethical guidelines of the University of Zurich, were plated at 100'000 cells per well in RPMI medium supplemented with 10% fetal calf serum (FCS) in 96 well plates and stimulated with either recombinant human IL-12 (Peprotech) or human IL-12Fc (SEQ ID 01). Both cytokines were normalized to each other according to concentrations derived from human IL-12p70 ELISA (Mabtech, #2455-1H-6). PBMCs were stimulated in the presence of 1µg/ml of a mouse IgG2a anti-human CD3 antibody (OKT3, Bio-X-cell). After two days of culture in 5% CO₂ and 37°C, supernatant was harvested and subjected to an anti-human IFN-γ ELISA (Mabtech, #3420-1H-6).

### Orthotopic glioma inoculation

Briefly, 6-10 week old mice were i.p. injected with Fluniximin (Biokema, 5mg/kg body weight) before being anaesthesized with 3-5% isoflurane (Minrad) in an induction chamber. Their heads were shaved with an electric hair-trimmer. After being mounted onto a stereotactic frame (David Kopf Instruments), the animals' scalp was disinfected with 10% iodine solution and a skin incision was made along the midline. Anaesthesia on the stereotactic frame was maintained at 3% isoflurane delivered through a nose adaptor (David Kopf Instruments). Subsequently, a blunt ended syringe (Hamilton, 75N, 26s/2"/2, 5µl) was mounted on a microinjection pump on the manipulator arm and placed 1.5mm lateral and 1 mm frontal of bregma. The needle was lowered into the manually drilled burr hole at a depth of 4mm below the dura surface and retracted 1 mm to form a small reservoir. Using the microinjection pump (UMP-3, World Precision Instruments Inc.) 2x10⁴ cells were injected in a volume of 2µl at 1 µl/min. After leaving the needle in place for 2min, it was retracted at 1 mm/min. The burr hole was closed with bone wax (Aesculap, Braun) and the scalp wound was sealed with tissue glue (Indermil, Henkel).

### In vivo bioluminescent imaging

Tumour bearing mice were carefully weighed, anaesthesized with isoflurane (2-3%) and injected with D-Luciferin (150mg/kg body weight, CaliperLifesciences). Animals were transferred to the dark chamber of a Xenogen IVIS 100 (CaliperLifesciences) imaging system, anaesthesia was maintained at 2% isoflurane via nosecones. 10min after injection luminescence was recorded. Data was subsequently analyzed using Living Image 2.5 software (CaliperLifesciences). A circular region of interest (ROI; 1.46cm Ø) was defined around the animals' head and photon flux of this region was read out and plotted.

### Treatment of established gliomas

At d21 after implantation of the glioma cells, the tumour bearing animals were evenly distributed among experimental groups based on their ROI-photon flux. Animals with an ROI flux of less than 1x10⁵ p/s were considered as non-takers and excluded. 40-48h prior to implantation (2 days before beginning of treatment), osmotic pumps (Model 2004, 0.25µl/h; Alzet) were filled with murine IL-12Fc (SEQ ID 02, 8.33 ng/µl in PBS) or PBS alone and primed at 37°C in PBS. Immediately prior to surgery, mice were injected with Fluniximini.p. (Biokema, 5mg/kg body weight). Mice were anaesthesized with 3-5% isoflurane, the scalp was disinfected and a midline incision was made. The previous burr hole of the glioma injection was located, the bone wax and periost removed and the pump placed into a skin pouch formed at the animal's back. The infusion cannula was lowered through the burr hole 3mm into the putative center of the tumour. The cannula was connected to the pump (brain infusion kit III 1-3mm, Alzet) via a silicon tube and held in place with cyanoacrylate adhesive. The skin was sutured with a 4-0 nylon thread. Following surgery, mice were treated for 3 days with 0.1% (v/v) Borgal (Intervet) in the drinking water. Pumps were explanted at day 49. Five doses of anti mouse-CTLA-4 mouse-IgG2b antibodies (clone 9D9, bio-X-cell; Peggs et al.; J Exp Med 206, 1717-1725 (2009)) or an equivalent volume of PBS were i.p. injected at days 22 (200µg), 26 (100µg), 29 (100µg), 35 (100µg) and 42 (100µg).

Alternatively, animals received anti-mouse-PD-1 rat IgG2a (clone RMP1-14, bio-X-cell) or rat IgG2a isotype control antibodies (clone 2A3, bio-X-cell) for the experiment depicted in figure 9. Dosing schedule and application route was identical with the experiment depicted in figure 7. For treatment of established B16-F10 derived brain tumours, pumps were implanted at day 5 post injection, anti mouse-CTLA-4 mouse-IgG2b antibodies (clone 9D9, bio-X-cell; Peggs et al.; J Exp Med 206, 1717-1725 (2009)) or an equivalent volume of PBS were i.p. injected at days 6 (200µg), 11 (100µg), 13 (100µg) and 19 (100µg).

### Survival analysis of tumour bearing animals

Tumour bearing animals were monitored by BLI, checked for neurological symptoms and weighed weekly until day 21 post glioma inoculation. GI261 Fc animals exhibiting an ROI flux of less than 1x10⁵ p/s at day 21 were considered as non or slow-tumour takers and excluded from the survival analysis (5-10%). From day 21 onwards animals were checked daily. Animals that showed symptoms as apathy, severe hunchback posture and/or weight loss of over 20% of peak weight were euthanized. B16-F10 tumour bearing mice were scored daily starting at day 5 until the end of experiment according to the same scheme.

### Histology

For histology, animals were euthanized with CO₂, transcardially perfused with ice-cold PBS and decapitated. Whole brains were carefully isolated, fixed in 4% Formalin, embedded in Paraffin and 3µm sections were processed for HE staining and/or immunohistochemistry to detect F4/80 (BM8; BMA biomedicals). Primary antibodies were detected with HorseRadish Peroxidase- coupled secondary antibodies. Staining was visualized with 3,3'-Diaminobenzidin (DAB) as the HRP substrate. Pictures were generated using an Olympus BX41 light microscope equipped with an Olympus ColorViewlllu camera and Olympus cell^B image acquisition software. Overviews of whole brains slices were cropped using Adobe Photoshop CS3.

### Statistical analysis

For statistical analysis of Kaplan-Meier survival curves, a Log-rank (Mantel-Cox) Test was used to calculate the p-values indicated in respective figures. P values of less than 0.05 were considered statistically significant. Analysis was performed with GraphPad Prism version 5.0a for Mac OSX (GraphPad Software Inc).

### Example 1: Intratumoural expression of IL-12Fc promotes clearance of experimental gliomas

We have designed and cloned a fusion protein consisting of the p40 subunit of human IL-12 linked via a flexible peptide linker to the p35 subunit. This single chain construct was then fused to the constant region of human IgG4 heavy chain (Fig. 1A). We termed this human single chain fusion protein IL-12Fc (SEQ ID 01.) We expressed this protein in HEK293 human embryonic kidney cells and detected a dimeric as well as a monomeric form under native conditions. Under reducing conditions only the monomeric form is detectable (Fig. 1B). IL-12Fc (SEQ ID 01) has similar functional properties as commercially available heterodimeric IL-12 (purchased from Peprotech). To determine if IL-12Fc (SEQ ID 01) could be suitable to overcome the local immunosuppressive environment induced by gliomas and to shed light on the effector mechanisms involved, we expressed a murine version (Belladonna et al. J Immunol 168, 5448-5454 (2002), IL-12Fc (SEQ ID 02)) of this cytokine in GI261 mouse glioma cells. To measure intracranial tumour growth non-invasively via bioluminescence imaging (BLI) we first generated a GI261 line that constitutively expresses *photinus pyralis* luciferase. We termed this cell line GI261-luc. We next modified this cell line to continuously release a fusion protein of IL-12 and the crystallizable fragment of mouse immunoglobulin G3 (IL-12Fc; SEQ ID 02, 09) or Fc (SEQ ID 08) alone as a control (termed 'GI261 IL-12Fc'and 'GI261 Fc', respectively). The chosen murine protein sequence of the fusion construct is homologous to the human variant (SEQ ID 01) and consists of the subunits p40 and p35 of IL-12 which are connected by a linker (G4S)₃ and the subunits CH2, CH3 and the last six amino acids of CH1 of the crystallizable fragment of IgG3, whereas CH1 and CH2 are connected by a hinge region. Vectors for expression of the control fragment and the IL-12 fusion construct are depicted in SEQ ID 08 and 09, respectively. The intention of using fusion proteins rather than the recombinant cytokine was to see whether they would exhibit improved pharmacokinetics. We confirmed secretion of IL-12Fc (SEQ ID 02) by ELISA for the subunits p40 and p70. We further confirmed expression of the Fc tail by RT-PCR. When implanted intracranially into the right striatum, luminescence readings and tumour volume as assessed by stereologic methods showed a robust correlation (data not shown).

We next implanted GI261 IL-12Fc and Fc into the right striatum of syngenic C57BI/6 mice and followed tumour growth via non invasive bioluminescence imaging (BLI). After an initial increase in luminescence all groups showed a depression around day 14 post injection. Animals bearing Fc-expressing tumours exhibited a steep increase in BLI and soon reached withdrawal criteria, sometimes even before day 35 post injection. In contrast, BLI-readings for animals that had been injected with IL-12Fc expressing GI261 tumours dropped to levels close to the detection limit at day 21 onwards (data not shown). In agreement with this observation, we could only detect a residual tumour in some animals in this group, while Fc control-injected animals showed robust tumour formation when analyzed histologically (Fig. 2). When we followed animals that had been implanted with GI261 IL-12Fc or GI261 Fc cells for up to 90 days, we observed rejection of the tumour in a high proportion of mice bearing IL-12Fc secreting tumours after an initial establishment (
Fig. 3).

### Example 2: T-cells are the major effector cell type of IL-12Fc mediated glioma rejection.

To confirm that the secretion of IL-12Fc by GI261 IL-12Fc acts on the host rather than the tumour cells themselves, we observed the growth of GI261 IL-12Fc and GI261 Fc to be the same in mice lacking the receptor to IL-12. The unbridled growth of GI261 IL-12 in *IL-12rβ2*^{*-*/*-*} animals demonstrates that IL-12Fc acts specifically on a cell type in the recipient mouse (data not shown). T and NK cells are among the most prominent IL-12 responsive leukocytes. To systematically test the functional relevance of the IL-12Fc mediated influx of these cells, we challenged a series of mouse mutants with intracranial GI261 IL-12Fc. We implanted GI261 IL-12Fc cells in mice that lack T and B cells (*Rag1*^{*-*/*-*}) or conventional Nk-cells (*II-15ra*^{*-*/*-*}) or in mice lacking both T-, B-, Nk-cells and lymphoid tissue inducer-like cells (*Rag2*^{*-*/}*⁻ II2rg*^{*-*/*-*}) (Fig. 4A). After an initial lag phase until day 14 after injection, all groups exhibited a strong increase in luminescence until day 28, reflecting strong tumour growth. Between days 28 and 42 most of the animals succumbed to the tumours. Only wt and *II-15ra*^{*-*/*-*} mice were able to control the tumour and show a significantly prolonged survival compared to *Rag2*^{*-*/}*⁻ II2rg*^{*-*/*-*} and *Rag1*^{*-*/*-*} animals. While T or B-cells appeared to be crucial for IL-12Fc mediated glioma rejection, the ability of *II-15ra*^{*-*/*-*} mice to reject GI261 IL-12 indicates that NK cells were largely expendable.

We next investigated the contribution of CD4- and CD8 positive T-cells using MHCII (*Ia*(*b*)^{*-*/*-*}) and MHCI (*β2m*^{*-*/*-*}) deficient mice. In contrast to wt mice, *Ia(b)*^{*-*/*-*} mice lacking CD4 T cells could not control GI261 IL-12Fc tumours, and *β*2m^{*-*/*-*} mice succumbed to the glioma shortly afterwards (Fig.4B). The survival in both mutant groups was shortened compared to the wildtype group. These data clearly demonstrate that IL-12Fc mediated tumour rejection is dependent on the activity of T cells including helper T cells and CTLs.

### Example 3: The antitumoural memory response is independent of ectopically expressed IL-12Fc.

To further investigate the character of the T-cell dependent tumour control, we tested the surviving wt animals that had been previously challenged with GI261 IL-12Fc cells for T cell memory formation (Fig. 5). The animals were treated as described in Fig. 3 / example 1. In contrast to the primary challenge, we now injected GI261 Fc cells into the contralateral hemisphere of survivors or naïve wt animals. We observed a rapid rejection of the control tumours within days. While the measured luminescence at day 1 suggested identical seeding across the two groups, only the naïve mice exhibited a measurable signal at day 7 onwards, suggesting a rapid and effectively clearing anti-glioma memory response now independent of ectopically expressed pro-inflammatory cytokines.

### Example 4: CTLs are the main effector cells of IL-12Fc-mediated glioma rejection.

It is well established that IL-12 polarizes naive T-cells to adopt a T_{H}1 phenotype (Trinchieri, Nat Rev Immunol 3, 133-146 (2003)). To shed further light on the mechanistic underpinnings underlying the IL-12 induced rejection of experimental glioma, we challenged mice deficient in the TH₁ hallmark cytokine IFN-γ (*Ifng*^{*-*/*-*}) with IL-12Fc expressing GI261 cells (Fig. 6A). The animals were treated as described in Fig. 3/ Example 1. To our surprise we observed a similar tumour rejection as in wt animals, suggesting that the mechanism of rejection is independent of IFN-γ. Conversely, IL-12 also stimulates the cytotoxic activity of CTLs. When we analyzed the role of Perforin, a cytolytic molecule primarily expressed on CD8⁺ CTLs and Nk-cells, we observed a clear difference in survival curves. (Fig. 6B). Perforin is a cytolytic molecule primarily expressed by CD8⁺ CTLs and NK cells but also CD4⁺ T-cells. To further investigate the mechanism of IL-12Fc induced rejection of glioma, perforin-deficient mice (*prfr1*^{*-*/*-*}) were challenged with IL-12Fc expressing GI261 cells. In contrast to *Ifng*^{*-*/*-*}, Perforin deficient animals (*prfr1*^{*-*/*-*}) were not able to control the tumour. This further supports the notion that CTLs are the main effector cells of IL-12Fc mediated glioma rejection. A clear difference in the survival curves of wt and *prf1*^{*-*/*-*}was observed.

### Example 5: Local administration of IL-12Fc in combination with systemic CTLA-4 blockade is effective against advanced stage experimental gliomas

To further boost and prolong the activated phenotype of T-cells, we blocked the co-inhibitory molecule CTLA-4 via neutralizing antibodies in the next set of experiments. IL-12 was administered locally to mice with advanced stage tumours. Treatment was administered to animals that had been challenged with GI261 Fc 21 days before and that already exhibited strong bioluminescence signals, indicating an advanced stage of glioma growth. Local treatment: At day 21, osmotic minipumps delivering 50 ng IL-12Fc/day (or PBS) into the tumour were implanted into glioma bearing animals. After 28 days (day 49 after tumour injection) the empty pumps were explanted from surviving animals. Systemic treatment: At day 22, tumour bearing animals received 200 µg αCTLA-4 mouse IgG2b (9D9) or PBS i.p. Treatment was sustained with 100 µg αCTLA-4 at days 26, 29,35 and 42 (Fig. 7). Neither IL-12Fc, nor anti-CTLA-4 alone conferred any significant survival advantage. Strikingly, the combination of local IL-12Fc administration directly into the tumour site in combination with systemic CTLA-4 blockade led to a full remission of the tumour (Fig. 8). 90 days after inoculation, histologic assessment of the brain tissue of surviving animals did not show any signs of demyelination or infiltrates. Local IL-12Fc administration in combination with systemic PD-1 blockade also led to a significant increase in surviving animals, the frequency was however lower than with systemic CTLA-4 blockade (Fig. 9). The above described combination therapy (Fig. 7) confers a significant survival advantage even in the case of intracranial growth of B16-F10 syngeneic murine melanoma cells (Fig. 10). This is not a perfect model for secondary brain tumours since it is skipping various steps of metastasis formation. Even in this more aggressive situation the combination treatment prolongs survival.

Preventive treatment of tumours in preclinical models may allow the study of immunological mechanisms and the interactions between tumour cells and tumour microenvironment. However, preventive therapy is of limited clinical relevance in the translation to treat cancer patients. We thus decided to choose an exceptionally late timepoint for intervention in a progressing and aggressive disease model. To closely mimic a clinical situation, we allowed the tumour to progress to a size that is highly likely to cause significant neurological symptoms in humans. Here, monotherapy with locally applied (intratumoural) IL-12 had a minimal albeit significant survival effect. We already observed a weak synergistic effect when we combined systemic IL-12 treatment with systemic CTLA-4 blockade. When local IL-12 infusion was combined with systemic CTLA-4 blockade, the anti-glioma effect was striking.

## Claims

1. A combination medicament, comprising
- an IL-12 polypeptide, or a nucleic acid expression vector comprising a sequence encoding said IL-12 polypeptide, and
- a non-agonist CTLA-4 ligand selected from the group consisting of an antibody, an antibody fragment, and an antibody-like molecule, wherein said non-agonist CTLA-4 ligand is capable of binding to CTLA-4 with a dissociation constant of at least 10-7 M⁻¹, and wherein said non-agonist CTLA-4 ligand is provided as a dosage form for systemic delivery,
for use in the therapy of a malignant neoplastic disease, wherein
- said IL-12 polypeptide, or said nucleic acid expression vector, is provided by administration into a tumour, into the vicinity of a tumour, or to the lymph node associated with a tumour.

2. A combination medicament for use in the therapy of a malignant neoplastic disease according to claim 1, wherein said IL-12 polypeptide comprises
a. a polypeptide sequence at least 95% identical to the sequence of human p35 (SEQ ID 05), and
b. a polypeptide sequence at least 95% identical to the sequence of human p40 (SEQ ID 06).

3. A combination medicament for use in the therapy of a malignant neoplastic disease according to claim 2, wherein said IL-12 polypeptide comprises an immunoglobulin G crystallisable fragment.

4. A combination medicament for use in the therapy of a malignant neoplastic disease according to claim 2 or 3, wherein said IL-12 polypeptide comprises a human immunoglobulin G subgroup 4 crystallisable fragment.

5. A combination medicament for use in the therapy of a malignant neoplastic disease according to claim 4, wherein said IL-12 polypeptide comprises
a. an immunoglobulin G crystallisable fragment and a recombinant or synthetic human IL-12 sequence, or
b. a sequence at least 95% identical to SEQ ID 01.

6. A combination medicament for use in the therapy of a malignant neoplastic disease according to any one of the above claims, wherein said non-agonist CTLA-4 ligand is a gamma immunoglobulin that binds to CTLA-4.

7. A combination medicament for use in the therapy of a malignant neoplastic disease according to any of the above claims, wherein said IL-12 polypeptide is provided as a dosage form for intratumoural injection.

8. A combination medicament for use in the therapy of a malignant neoplastic disease according to any of the above claims, wherein said non-agonist CTLA-4 ligand is provided as a dosage form for intravenous injection or local application.

9. A combination medicament according to at least one of claims 1 to 8 for use in a method of therapy of glioma, glioblastoma multiforme, meningioma, secondary brain cancer, brain metastases, melanoma, pancreatic cancer, lung cancer, prostate cancer or bladder cancer.

10. A combination medicament according to claim 1, wherein
- said IL-12 polypeptide is a fusion protein comprising the amino acid of human p40, the amino acid sequence of human p35 and the crystallisable fragment of human IgG4,
- said IL-12 polypeptide is provided as a dosage form for intratumoural delivery, and wherein
- said non-agonist CTLA-4 ligand is an immunoglobulin G provided as a dosage form for systemic delivery,
for use in the treatment of malignant neoplastic disease, particularly glioma, glioblastoma multiforme, meningioma, secondary brain cancer, brain metastases, melanoma, pancreatic cancer, lung cancer, prostate cancer or bladder cancer.

11. A combination medicament for use in the therapy of a malignant neoplastic disease according to at least one of claims 1 to 8, wherein said nucleic acid expression vector is an adenovirus, an adeno-associated virus, a lentivirus or a herpesvirus.

## Patentansprüche

1. Kombinationsmedikament, umfassend
- ein IL-12-Polypeptid, oder einen Nukleinsäureexpressionsvektor, der eine Sequenz umfasst, die das IL-12-Polypeptid codiert, und
- einen nicht-agonistischen CTLA-4-Liganden, ausgewählt aus der Gruppe bestehend aus einem Antikörper, einem Antikörperfragment und einem antikörperartigen Molekül, wobei der nicht-agonistische CTLA-4-Ligand in der Lage ist, mit einer Dissoziationskonstante von mindestens 10-7 M⁻¹ an CTLA-4 zu binden, und wobei der nicht-agonistische CTLA-4-Ligand als eine Darreichungsform zur systemischen Abgabe bereitgestellt wird,
zur Verwendung in der Therapie einer malignen neoplastischen Erkrankung, wobei
- das IL-12-Polypeptid, oder der Nukleinsäureexpressionsvektor, durch Verabreichung in einen Tumor, in die Nähe eines Tumors, oder an den Lymphknoten, der mit einem Tumor assoziiert ist, bereitgestellt wird.

2. Kombinationsmedikament zur Verwendung in der Therapie einer malignen neoplastischen Erkrankung nach Anspruch 1, wobei das IL-12-Polypeptid
a. eine Polypeptidsequenz, die zu mindestens 95 % mit der Sequenz von humanem p35 (SEQ-ID 05) identisch ist, und
b. eine Polypeptidsequenz, die zu mindestens 95 % mit der Sequenz von humanem p40 (SEQ-ID 06) identisch ist
umfasst.

3. Kombinationsmedikament zur Verwendung in der Therapie einer malignen neoplastischen Erkrankung nach Anspruch 2, wobei das IL-12-Polypeptid ein Immunglobulin G kristallisierbares Fragment umfasst.

4. Kombinationsmedikament zur Verwendung in der Therapie einer malignen neoplastischen Erkrankung nach Anspruch 2 oder 3, wobei das IL-12-Polypeptid ein humanes Immunglobulin G Subgruppe 4 kristallisierbares Fragment umfasst.

5. Kombinationsmedikament zur Verwendung in der Therapie einer malignen neoplastischen Erkrankung nach Anspruch 4, wobei das IL-12-Polypeptid
a. ein Immunglobulin G kristallisierbares Fragment und eine rekombinante oder synthetische humane IL-12-Sequenz, oder
b. eine Sequenz, die zu mindestens 95 % mit SEQ-ID 01 identisch ist umfasst.

6. Kombinationsmedikament zur Verwendung in der Therapie einer malignen neoplastischen Erkrankung nach einem der vorstehenden Ansprüche, wobei der nicht-agonistische CTLA-4-Ligand ein Gamma-Immunglobulin ist, das an CTLA-4 bindet.

7. Kombinationsmedikament zur Verwendung in der Therapie einer malignen neoplastischen Erkrankung nach einem der vorstehenden Ansprüche, wobei das IL-12-Polypeptid als eine Darreichungsform zur intratumoralen Injektion bereitgestellt wird.

8. Kombinationsmedikament zur Verwendung in der Therapie einer malignen neoplastischen Erkrankung nach einem der vorstehenden Ansprüche, wobei der nicht-agonistische CTLA-4-Ligand als eine Darreichungsform zur intravenösen Injektion oder lokalen Anwendung bereitgestellt wird.

9. Kombinationsmedikament nach mindestens einem der Ansprüche 1 bis 8 zur Verwendung in einem Therapieverfahren von Gliom, Glioblastoma multiforme, Meningiom, sekundärem Gehirnkrebs, Gehirnmetastasen, Melanom, Bauchspeicheldrüsenkrebs, Lungenkrebs, Prostatakrebs oder Blasenkrebs.

10. Kombinationsmedikament nach Anspruch 1, wobei
- das IL-12-Polypeptid ein Fusionsprotein ist, das die Aminosäure von humanem p40, die Aminosäuresequenz von humanem p35 und das kristallisierbare Fragment von humanem IgG4 umfasst,
- das IL-12-Polypeptid als eine Darreichungsform zur intratumoralen Abgabe bereitgestellt wird, und wobei
- der nicht-agonistische CTLA-4-Ligand ein Immunglobulin G ist, das als eine Darreichungsform zur systemischen Abgabe bereitgestellt wird,
zur Verwendung in der Behandlung von maligner neoplastischer Erkrankung, insbesondere Gliom, Glioblastoma multiforme, Meningiom, sekundärem Gehirnkrebs, Gehirnmetastasen, Melanom, Bauchspeicheldrüsenkrebs, Lungenkrebs, Prostatakrebs oder Blasenkrebs.

11. Kombinationsmedikament zur Verwendung in der Therapie einer malignen neoplastischen Erkrankung nach mindestens einem der Ansprüche 1 bis 8, wobei der Nukleinsäureexpressionsvektor ein Adenovirus, ein Adeno-assoziiertes Virus, ein Lentivirus oder ein Herpesvirus ist.

## Revendications

1. Combinaison médicamenteuse, comprenant :
- un polypeptide IL-12 ou un vecteur d'expression d'acide nucléique comprenant une séquence codant pour ledit polypeptide IL-12, et
- un ligand de CTLA-4 non agoniste sélectionné dans le groupe constitué d'un anticorps, d'un fragment d'anticorps et d'une molécule de type anticorps, dans laquelle ledit ligand de CTLA-4 non agoniste est capable de se lier à du CTLA-4 avec une constante de dissociation d'au moins 10-7 M⁻¹ et dans laquelle ledit ligand de CTLA-4 non agoniste est fourni sous une forme posologique pour administration systémique,
pour utilisation dans la thérapie d'une maladie néoplasique maligne, dans laquelle :
- ledit polypeptide IL-12 ou ledit vecteur d'expression d'acide nucléique est fourni par administration à une tumeur, au voisinage d'une tumeur ou au ganglion lymphatique associé à une tumeur.

2. Combinaison médicamenteuse pour utilisation dans la thérapie d'une maladie néoplasique maligne selon la revendication 1, dans laquelle ledit polypeptide IL-12 comprend :
a. une séquence de polypeptide au moins à 95 % identique à la séquence du p35 humain (SEQ ID 05) et
b. une séquence de polypeptide au moins à 95 % identique à la séquence du p40 humain (SEQ ID 06).

3. Combinaison médicamenteuse pour utilisation dans la thérapie d'une maladie néoplasique maligne selon la revendication 2, dans laquelle ledit polypeptide IL-12 comprend un fragment cristallisable d'immunoglobuline G.

4. Combinaison médicamenteuse pour utilisation dans la thérapie d'une maladie néoplasique maligne selon la revendication 2 ou 3, dans laquelle ledit polypeptide IL-12 comprend un fragment humain cristallisable du sous-groupe 4 de l'immunoglobuline G.

5. Combinaison médicamenteuse pour utilisation dans la thérapie d'une maladie néoplasique maligne selon la revendication 4, dans laquelle ledit polypeptide IL-12 comprend :
a. un fragment cristallisable d'immunoglobuline G et une séquence IL-12 recombinante ou synthétique humaine ou
b. une séquence au moins à 95 % identique à la SEQ ID 01.

6. Combinaison médicamenteuse pour utilisation dans la thérapie d'une maladie néoplasique maligne selon l'une quelconque des revendications précédentes, dans laquelle ledit ligand de CTLA-4 non agoniste est une gamma immunoglobuline qui se lie à du CTLA-4.

7. Combinaison médicamenteuse pour utilisation dans la thérapie d'une maladie néoplasique maligne selon l'une quelconque des revendications précédentes, dans laquelle ledit polypeptide IL-12 est fourni sous une forme posologique pour injection intratumorale.

8. Combinaison médicamenteuse pour utilisation dans la thérapie d'une maladie néoplasique maligne selon l'une quelconque des revendications précédentes, dans laquelle ledit ligand de CTLA-4 non agoniste est fourni sous une forme posologique pour injection intraveineuse ou application locale.

9. Combinaison médicamenteuse selon au moins l'une quelconque des revendications 1 à 8 pour utilisation dans un procédé de thérapie de gliome, de glioblastome multiforme, de méningiome, de cancer du cerveau secondaire, de métastases du cerveau, de mélanome, de cancer du pancréas, de cancer du poumon, du cancer de la prostate ou du cancer de la vessie.

10. Combinaison médicamenteuse selon la revendication 1, dans laquelle :
- ledit polypeptide IL-12 est une protéine de fusion comprenant l'acide aminé du p40 humain, la séquence d'acide aminé du p35 humain et le fragment cristallisable du l'IgG4 humain,
- ledit polypeptide IL-12 est fourni sous une forme posologique pour administration intratumorale et dans laquelle :
- ledit ligand de CTLA-4 non agoniste est une immunoglobuline G fournie sous une forme posologique pour administration systémique,
pour utilisation dans le traitement d'une maladie néoplasique maligne, en particulier le gliome, le glioblastome multiforme, le méningiome, le cancer du cerveau secondaire, les métastases du cerveau, le mélanome, le cancer du pancréas, le cancer du poumon, le cancer de la prostate et le cancer de la vessie.

11. Combinaison médicamenteuse pour utilisation dans la thérapie d'une maladie néoplasique maligne selon au moins l'une quelconque des revendications 1 à 8, dans laquelle ledit vecteur d'expression d'acide nucléique est un adénovirus, un virus adéno-associé, un lentivirus ou un herpèsvirus.


### SEQUENCE LISTING

<110> Universitaet Zuerich
<120> Combination medicament comprising IL-12 and an agent for blockade of T-cell inhibitory molecules for tumour therapy
<130> uz117wo
<160> 9
<170> PatentIn version 3.5
<210> 1
   <211> 775
   <212> PRT
   <213> artificial
<220>
   <223> fusion between human IL-12 and IgG4 Fc
<400> 1
   Met Cys His Gln Gln Leu Val Ile Ser Trp Phe Ser Leu Val Phe Leu
   1               5                   10                  15
   Ala Ser Pro Leu Val Ala Ile Trp Glu Leu Lys Lys Asp Val Tyr Val
               20                  25                  30
   Val Glu Leu Asp Trp Tyr Pro Asp Ala Pro Gly Glu Met Val Val Leu
           35                  40                  45
   Thr Cys Asp Thr Pro Glu Glu Asp Gly Ile Thr Trp Thr Leu Asp Gln
       50                  55                  60
   Ser Ser Glu Val Leu Gly Ser Gly Lys Thr Leu Thr Ile Gln Val Lys
   65                  70                  75                  80
   Glu Phe Gly Asp Ala Gly Gln Tyr Thr Cys His Lys Gly Gly Glu Val
                   85                  90                  95
   Leu Ser His Ser Leu Leu Leu Leu His Lys Lys Glu Asp Gly Ile Trp
               100                 105                 110
   Ser Thr Asp Ile Leu Lys Asp Gln Lys Glu Pro Lys Asn Lys Thr Phe
           115                 120                 125
   Leu Arg Cys Glu Ala Lys Asn Tyr Ser Gly Arg Phe Thr Cys Trp Trp
       130                 135                 140
   Leu Thr Thr Ile Ser Thr Asp Leu Thr Phe Ser Val Lys Ser Ser Arg
   145                 150                 155                 160
   Gly Ser Ser Asp Pro Gln Gly Val Thr Cys Gly Ala Ala Thr Leu Ser
                   165                 170                 175
   Ala Glu Arg Val Arg Gly Asp Asn Lys Glu Tyr Glu Tyr Ser Val Glu
               180                 185                 190
   Cys Gln Glu Asp Ser Ala Cys Pro Ala Ala Glu Glu Ser Leu Pro Ile
           195                 200                 205
   Glu Val Met Val Asp Ala Val His Lys Leu Lys Tyr Glu Asn Tyr Thr
       210                 215                 220
   Ser Ser Phe Phe Ile Arg Asp Ile Ile Lys Pro Asp Pro Pro Lys Asn
   225                 230                 235                 240
   Leu Gln Leu Lys Pro Leu Lys Asn Ser Arg Gln Val Glu Val Ser Trp
                   245                 250                 255
   Glu Tyr Pro Asp Thr Trp Ser Thr Pro His Ser Tyr Phe Ser Leu Thr
               260                 265                 270
   Phe Cys Val Gln Val Gln Gly Lys Ser Lys Arg Glu Lys Lys Asp Arg
           275                 280                 285
   Val Phe Thr Asp Lys Thr Ser Ala Thr Val Ile Cys Arg Lys Asn Ala
       290                 295                 300
   Ser Ile Ser Val Arg Ala Gln Asp Arg Tyr Tyr Ser Ser Ser Trp Ser
   305                 310                 315                 320
   Glu Trp Ala Ser Val Pro Cys Ser Gly Gly Gly Gly Ser Gly Gly Gly
                   325                 330                 335
   Gly Ser Gly Gly Gly Gly Ser Arg Asn Leu Pro Val Ala Thr Pro Asp
               340                 345                 350
   Pro Gly Met Phe Pro Cys Leu His His Ser Gln Asn Leu Leu Arg Ala
           355                 360                 365
   Val Ser Asn Met Leu Gln Lys Ala Arg Gln Thr Leu Glu Phe Tyr Pro
       370                 375                 380
   Cys Thr Ser Glu Glu Ile Asp His Glu Asp Ile Thr Lys Asp Lys Thr
   385                 390                 395                 400
   Ser Thr Val Glu Ala Cys Leu Pro Leu Glu Leu Thr Lys Asn Glu Ser
                   405                 410                 415
   Cys Leu Asn Ser Arg Glu Thr Ser Phe Ile Thr Asn Gly Ser Cys Leu
   420 425 430
   Ala Ser Arg Lys Thr Ser Phe Met Met Ala Leu Cys Leu Ser Ser Ile
   435 440 445
   Tyr Glu Asp Leu Lys Met Tyr Gln Val Glu Phe Lys Thr Met Asn Ala
   450 455 460
   Lys Leu Leu Met Asp Pro Lys Arg Gln Ile Phe Leu Asp Gln Asn Met
   465 470 475 480
   Leu Ala Val Ile Asp Glu Leu Met Gln Ala Leu Asn Phe Asn Ser Glu
   485 490 495
   Thr Val Pro Gln Lys Ser Ser Leu Glu Glu Pro Asp Phe Tyr Lys Thr
   500 505 510
   Lys Ile Lys Leu Cys Ile Leu Leu His Ala Phe Arg Ile Arg Ala Val
   515 520 525
   Thr Ile Asp Arg Val Met Ser Tyr Leu Asn Ala Ser Lys Val Asp Lys
   530 535 540
   Arg Val Glu Ser Lys Tyr Gly Pro Pro Cys Pro Ser Cys Pro Ala Pro
   545 550 555 560
   Glu Phe Leu Gly Gly Pro Ser Val Phe Leu Phe Pro Pro Lys Pro Lys
   565 570 575
   Asp Thr Leu Met Ile Ser Arg Thr Pro Glu Val Thr Cys Val Val Val
   580 585 590
   Asp Val Ser Gln Glu Asp Pro Glu Val Gln Phe Asn Trp Tyr Val Asp
   595 600 605
   Gly Val Glu Val His Asn Ala Lys Thr Lys Pro Arg Glu Glu Gln Phe
   610 615 620
   Asn Ser Thr Tyr Arg Val Val Ser Val Leu Thr Val Leu His Gln Asp
   625 630 635 640
   Trp Leu Asn Gly Lys Glu Tyr Lys Cys Lys Val Ser Asn Lys Gly Leu
   645 650 655
   Pro Ser Ser Ile Glu Lys Thr Ile Ser Lys Ala Lys Gly Gln Pro Arg
   660 665 670
   Glu Pro Gln Val Tyr Thr Leu Pro Pro Ser Pro Glu Glu Met Thr Lys
   675 680 685
   Asn Gln Val Ser Leu Thr Cys Leu Val Lys Gly Phe Tyr Pro Ser Asp
   690 695 700
   Ile Ala Val Glu Trp Glu Ser Asn Gly Gln Pro Glu Asn Asn Tyr Lys
   705 710 715 720
   Thr Thr Pro Pro Val Leu Asp Ser Asp Gly Ser Phe Phe Leu Tyr Ser
   725 730 735
   Arg Leu Thr Val Asp Lys Ser Arg Trp Gln Glu Gly Asn Val Phe Ser
   740 745 750
   Cys Ser Val Met His Glu Ala Leu His Asn His Tyr Thr Gln Lys Ser
   755 760 765
   Leu Ser Leu Ser Leu Gly Lys
   770 775
<210> 2
   <211> 781
   <212> PRT
   <213> Artificial Sequence
<220>
   <223> murine IL-12 IgG3 Fc fusion construct
<400> 2
   Met Cys Pro Gln Lys Leu Thr Ile Ser Trp Phe Ala Ile Val Leu Leu
   1 5 10 15
   Val Ser Pro Leu Met Ala Met Trp Glu Leu Glu Lys Asp Val Tyr Val
   20 25 30
   Val Glu Val Asp Trp Thr Pro Asp Ala Pro Gly Glu Thr Val Asn Leu
   35 40 45
   Thr Cys Asp Thr Pro Glu Glu Asp Asp Ile Thr Trp Thr Ser Asp Gln
   50 55 60
   Arg His Gly Val Ile Gly Ser Gly Lys Thr Leu Thr Ile Thr Val Lys
   65 70 75 80
   Glu Phe Leu Asp Ala Gly Gln Tyr Thr Cys His Lys Gly Gly Glu Thr
   85 90 95
   Leu Ser His Ser His Leu Leu Leu His Lys Lys Glu Asn Gly Ile Trp
               100                 105                 110
   Ser Thr Glu Ile Leu Lys Asn Phe Lys Asn Lys Thr Phe Leu Lys Cys
           115                 120                 125
   Glu Ala Pro Asn Tyr Ser Gly Arg Phe Thr Cys Ser Trp Leu Val Gln
       130                 135                 140
   Arg Asn Met Asp Leu Lys Phe Asn Ile Lys Ser Ser Ser Ser Ser Pro
   145                 150                 155                 160
   Asp Ser Arg Ala Val Thr Cys Gly Met Ala Ser Leu Ser Ala Glu Lys
                   165                 170                 175
   Val Thr Leu Asp Gln Arg Asp Tyr Glu Lys Tyr Ser Val Ser Cys Gln
               180                 185                 190
   Glu Asp Val Thr Cys Pro Thr Ala Glu Glu Thr Leu Pro Ile Glu Leu
           195                 200                 205
   Ala Leu Glu Ala Arg Gln Gln Asn Lys Tyr Glu Asn Tyr Ser Thr Ser
       210                 215                 220
   Phe Phe Ile Arg Asp Ile Ile Lys Pro Asp Pro Pro Lys Asn Leu Gln
   225                 230                 235                 240
   Met Lys Pro Leu Lys Asn Ser Gln Val Glu Val Ser Trp Glu Tyr Pro
                   245                 250                 255
   Asp Ser Trp Ser Thr Pro His Ser Tyr Phe Ser Leu Lys Phe Phe Val
               260                 265                 270
   Arg Ile Gln Arg Lys Lys Glu Lys Met Lys Glu Thr Glu Glu Gly Cys
           275                 280                 285
   Asn Gln Lys Gly Ala Phe Leu Val Glu Lys Thr Ser Thr Glu Val Gln
       290                 295                 300
   Cys Lys Gly Gly Asn Val Cys Val Gln Ala Gln Asp Arg Tyr Tyr Asn
   305                 310                 315                 320
   Ser Ser Cys Ser Lys Trp Ala Cys Val Pro Cys Arg Val Arg Ser Gly
                   325                 330                 335
   Gly Gly Gly Ser Gly Gly Gly Gly Ser Gly Gly Gly Gly Ser Arg Val
               340                 345                 350
   Ile Pro Val Ser Gly Pro Ala Arg Cys Leu Ser Gln Ser Arg Asn Leu
           355                 360                 365
   Leu Lys Thr Thr Asp Asp Met Val Lys Thr Ala Arg Glu Lys Leu Lys
       370                 375                 380
   His Tyr Ser Cys Thr Ala Glu Asp Ile Asp His Glu Asp Ile Thr Arg
   385                 390                 395                 400
   Asp Gln Thr Ser Thr Leu Lys Thr Cys Leu Pro Leu Glu Leu His Lys
                   405                 410                 415
   Asn Glu Ser Cys Leu Ala Thr Arg Glu Thr Ser Ser Thr Thr Arg Gly
               420                 425                 430
   Ser Cys Leu Pro Pro Gln Lys Thr Ser Leu Met Met Thr Leu Cys Leu
           435                 440                 445
   Gly Ser Ile Tyr Glu Asp Leu Lys Met Tyr Gln Thr Glu Phe Gln Ala
       450                 455                 460
   Ile Asn Ala Ala Leu Gln Asn His Asn His Gln Gln Ile Ile Leu Asp
   465                 470                 475                 480
   Lys Gly Met Leu Val Ala Ile Asp Glu Leu Met Gln Ser Leu Asn His
                   485                 490                 495
   Asn Gly Glu Thr Leu Arg Gln Lys Pro Pro Val Gly Glu Ala Asp Pro
               500                 505                 510
   Tyr Arg Val Lys Met Lys Leu Cys Ile Leu Leu His Ala Phe Ser Thr
           515                 520                 525
   Arg Val Val Thr Ile Asn Arg Val Met Gly Tyr Leu Ser Ser Ala Leu
       530                 535                 540
   Ile Lys Arg Ile Glu Pro Arg Ile Pro Lys Pro Ser Thr Pro Pro Gly
   545                 550                 555                 560
   Ser Ser Cys Pro Pro Gly Asn Ile Leu Gly Gly Pro Ser Val Phe Ile
                   565                 570                 575
   Phe Pro Pro Lys Pro Lys Asp Ala Leu Met Ile Ser Leu Thr Pro Lys
               580                 585                 590
   Val Thr Cys Val Val Val Asp Val Ser Glu Asp Asp Pro Asp Val His
               595                 600                 605
   Val Ser Trp Phe Val Asp Asn Lys Glu Val His Thr Ala Trp Thr Gln
       610                 615                 620
   Pro Arg Glu Ala Gln Tyr Asn Ser Thr Phe Arg Val Val Ser Ala Leu
   625                 630                 635                 640
   Pro Ile Gln His Gln Asp Trp Met Arg Gly Lys Glu Phe Lys Cys Lys
                   645                 650                 655
   Val Asn Asn Lys Ala Leu Pro Ala Pro Ile Glu Arg Thr Ile Ser Lys
               660                 665                 670
   Pro Lys Gly Arg Ala Gln Thr Pro Gln Val Tyr Thr Ile Pro Pro Pro
           675                 680                 685
   Arg Glu Gln Met Ser Lys Lys Lys Val Ser Leu Thr Cys Leu Val Thr
       690                 695                 700
   Asn Phe Phe Ser Glu Ala Ile Ser Val Glu Trp Glu Arg Asn Gly Glu
   705                 710                 715                 720
   Leu Glu Gln Asp Tyr Lys Asn Thr Pro Pro Ile Leu Asp Ser Asp Gly
                   725                 730                 735
   Thr Tyr Phe Leu Tyr Ser Lys Leu Thr Val Asp Thr Asp Ser Trp Leu
               740                 745                 750
   Gln Gly Glu Ile Phe Thr Cys Ser Val Val His Glu Ala Leu His Asn
           755                 760                 765
   His His Thr Gln Lys Asn Leu Ser Arg Ser Pro Gly Lys
       770                 775                 780
<210> 3
   <211> 17
   <212> DNA
   <213> artificial
<220>
   <223> PCR primer
<400> 3
   acacacagcc tggacgc 17
<210> 4
   <211> 20
   <212> DNA
   <213> artificial
<220>
   <223> PCR primer
<400> 4
   catttgaact ccttgcccct 20
<210> 5
   <211> 219
   <212> PRT
   <213> homo sapiens
<400> 5
   Met Cys Pro Ala Arg Ser Leu Leu Leu Val Ala Thr Leu Val Leu Leu
   1               5                   10                  15
   Asp His Leu Ser Leu Ala Arg Asn Leu Pro Val Ala Thr Pro Asp Pro
               20                  25                  30
   Gly Met Phe Pro Cys Leu His His Ser Gln Asn Leu Leu Arg Ala Val
           35                  40                  45
   Ser Asn Met Leu Gln Lys Ala Arg Gln Thr Leu Glu Phe Tyr Pro Cys
       50                  55                  60
   Thr Ser Glu Glu Ile Asp His Glu Asp Ile Thr Lys Asp Lys Thr Ser
   65                  70                  75                  80
   Thr Val Glu Ala Cys Leu Pro Leu Glu Leu Thr Lys Asn Glu Ser Cys
                   85                  90                  95
   Leu Asn Ser Arg Glu Thr Ser Phe Ile Thr Asn Gly Ser Cys Leu Ala
               100                 105                 110
   Ser Arg Lys Thr Ser Phe Met Met Ala Leu Cys Leu Ser Ser Ile Tyr
           115                 120                 125
   Glu Asp Leu Lys Met Tyr Gln Val Glu Phe Lys Thr Met Asn Ala Lys
       130                 135                 140
   Leu Leu Met Asp Pro Lys Arg Gln Ile Phe Leu Asp Gln Asn Met Leu
   145                 150                 155                 160
   Ala Val Ile Asp Glu Leu Met Gln Ala Leu Asn Phe Asn Ser Glu Thr
                   165                 170                 175
   Val Pro Gln Lys Ser Ser Leu Glu Glu Pro Asp Phe Tyr Lys Thr Lys
               180                 185                 190
   Ile Lys Leu Cys Ile Leu Leu His Ala Phe Arg Ile Arg Ala Val Thr
   195 200 205
   Ile Asp Arg Val Met Ser Tyr Leu Asn Ala Ser
   210 215
<210> 6
   <211> 328
   <212> PRT
   <213> homo sapiens
<400> 6
   Met Cys His Gln Gln Leu Val Ile Ser Trp Phe Ser Leu Val Phe Leu
   1 5 10 15
   Ala Ser Pro Leu Val Ala Ile Trp Glu Leu Lys Lys Asp Val Tyr Val
   20 25 30
   Val Glu Leu Asp Trp Tyr Pro Asp Ala Pro Gly Glu Met Val Val Leu
   35 40 45
   Thr Cys Asp Thr Pro Glu Glu Asp Gly Ile Thr Trp Thr Leu Asp Gln
   50 55 60
   Ser Ser Glu Val Leu Gly Ser Gly Lys Thr Leu Thr Ile Gln Val Lys
   65 70 75 80
   Glu Phe Gly Asp Ala Gly Gln Tyr Thr Cys His Lys Gly Gly Glu Val
   85 90 95
   Leu Ser His Ser Leu Leu Leu Leu His Lys Lys Glu Asp Gly Ile Trp
   100 105 110
   Ser Thr Asp Ile Leu Lys Asp Gln Lys Glu Pro Lys Asn Lys Thr Phe
   115 120 125
   Leu Arg Cys Glu Ala Lys Asn Tyr Ser Gly Arg Phe Thr Cys Trp Trp
   130 135 140
   Leu Thr Thr Ile Ser Thr Asp Leu Thr Phe Ser Val Lys Ser Ser Arg
   145 150 155 160
   Gly Ser Ser Asp Pro Gln Gly Val Thr Cys Gly Ala Ala Thr Leu Ser
   165 170 175
   Ala Glu Arg Val Arg Gly Asp Asn Lys Glu Tyr Glu Tyr Ser Val Glu
   180 185 190
   Cys Gln Glu Asp Ser Ala Cys Pro Ala Ala Glu Glu Ser Leu Pro Ile
           195                 200                 205
   Glu Val Met Val Asp Ala Val His Lys Leu Lys Tyr Glu Asn Tyr Thr
       210                 215                 220
   Ser Ser Phe Phe Ile Arg Asp Ile Ile Lys Pro Asp Pro Pro Lys Asn
   225                 230                 235                 240
   Leu Gln Leu Lys Pro Leu Lys Asn Ser Arg Gln Val Glu Val Ser Trp
                   245                 250                 255
   Glu Tyr Pro Asp Thr Trp Ser Thr Pro His Ser Tyr Phe Ser Leu Thr
               260                 265                 270
   Phe Cys Val Gln Val Gln Gly Lys Ser Lys Arg Glu Lys Lys Asp Arg
           275                 280                 285
   Val Phe Thr Asp Lys Thr Ser Ala Thr Val Ile Cys Arg Lys Asn Ala
       290                 295                 300
   Ser Ile Ser Val Arg Ala Gln Asp Arg Tyr Tyr Ser Ser Ser Trp Ser
   305                 310                 315                 320
   Glu Trp Ala Ser Val Pro Cys Ser
                   325
<210> 7
   <211> 2328
   <212> DNA
   <213> artificial
<220>
   <223> expression construct, coding sequence for IL-12 IgG4 Fc fusion protein
<400> 7
   atgtgtcacc agcagttggt catctcttgg ttttccctgg tttttctggc atctcccctc      60
   gtggccatat gggaactgaa gaaagatgtt tatgtcgtag aattggattg gtatccggat     120
   gcccctggag aaatggtggt cctcacctgt gacacccctg aagaagatgg tatcacctgg     180
   accttggacc agagcagtga ggtcttaggc tctggcaaaa ccctgaccat ccaagtcaaa     240
   gagtttggag atgctggcca gtacacctgt cacaaaggag gcgaggttct aagccattcg     300
   ctcctgctgc ttcacaaaaa ggaagatgga atttggtcca ctgatatttt aaaggaccag     360
   aaagaaccca aaaataagac ctttctaaga tgcgaggcca agaattattc tggacgtttc     420
   acctgctggt ggctgacgac aatcagtact gatttgacat tcagtgtcaa aagcagcaga     480
   ggctcttctg acccccaagg ggtgacgtgc ggagctgcta cactctctgc agagagagtc     540
   agaggggaca acaaggagta tgagtactca gtggagtgcc aggaggacag tgcctgccca      600
   gctgctgagg agagtctgcc cattgaggtc atggtggatg ccgttcacaa gctcaagtat      660
   gaaaactaca ccagcagctt cttcatcagg gacatcatca aacctgaccc acccaagaac      720
   ttgcagctga agccattaaa gaattctcgg caggtggagg tcagctggga gtaccctgac      780
   acctggagta ctccacattc ctacttctcc ctgacattct gcgttcaggt ccagggcaag      840
   agcaagagag aaaagaaaga tagagtcttc acggacaaga cctcagccac ggtcatctgc      900
   cgcaaaaatg ccagcattag cgtgcgggcc caggaccgct actatagctc atcttggagc      960
   gaatgggcat ctgtgccctg cagtggaggc ggtggctcgg gcggtggtgg gtcgggtggc     1020
   ggcggatcca gaaacctccc cgtggccact ccagacccag gaatgttccc atgccttcac     1080
   cactcccaaa acctgctgag ggccgtcagc aacatgctcc agaaggccag acaaactcta     1140
   gaattttacc cttgcacttc tgaagagatt gatcatgaag atatcacaaa agataaaacc     1200
   agcacagtgg aggcctgttt accattggaa ttaaccaaga atgagagttg cctaaattcc     1260
   agagagacct ctttcataac taatgggagt tgcctggcct ccagaaagac ctcttttatg     1320
   atggccctgt gccttagtag tatttatgaa gacttgaaga tgtaccaggt ggagttcaag     1380
   accatgaatg caaagcttct gatggatcct aagaggcaga tctttctaga tcaaaacatg     1440
   ctggcagtta ttgatgagct gatgcaggcc ctgaatttca acagtgagac tgtgccacaa     1500
   aaatcctccc ttgaagaacc ggatttttat aaaactaaaa tcaagctctg catacttctt     1560
   catgctttca gaattcgggc agtgactatt gatagagtga tgagctatct gaatgcttcc     1620
   aaggtggaca agagagttga gtccaaatat ggtcccccat gcccatcatg cccagcacct     1680
   gagttcctgg ggggaccatc agtcttcctg ttccccccaa aacccaagga cactctcatg     1740
   atctcccgga cccctgaggt cacgtgcgtg gtggtggacg tgagccagga agaccccgag     1800
   gtccagttca actggtacgt ggatggcgtg gaggtgcata atgccaagac aaagccgcgg     1860
   gaggagcagt tcaacagcac gtaccgtgtg gtcagcgtcc tcaccgtcct gcaccaggac     1920
   tggctgaacg gcaaggagta caagtgcaag gtctccaaca aaggcctccc gtcctccatc     1980
   gagaaaacca tctccaaagc caaagggcag ccccgagagc cacaggtgta caccctgccc     2040
   ccatccccgg aggagatgac caagaaccag gtcagcctga cctgcctggt caaaggcttc     2100
   taccccagcg acatcgccgt ggagtgggag agcaatgggc agccggagaa caactacaag     2160
   accacgcctc ccgtgctgga ctccgacggc tccttcttcc tctacagcag gctaaccgtg     2220
   gacaagagca ggtggcagga ggggaatgtc ttctcatgct ccgtgatgca tgaggctctg     2280
   cacaaccact acacacagaa gagcctctcc ctgtctctgg gtaaatga                 2328
<210> 8
   <211> 10994
   <212> DNA
   <213> artificial
<220>
   <223> plasmid vector encoding Fc tag (murine)
<400> 8
   ctcgcagcaa agcaagatgt gtcctcagaa gctaaccatc tcctggtttg ccatcgtttt     60
   gctggtgtct ccactcatgg ccatgtggga gctggagaag cttatcaaga gaatcgagcc    120
   tagaataccc aagcccagta cccccccagg ttcttcatgc ccacctggta acatcttggg    180
   tggaccatcc gtcttcatct tccccccaaa gcccaaggat gcactcatga tctccctaac    240
   ccccaaggtt acgtgtgtgg tggtggatgt gagcgaggat gacccagatg tccatgtcag    300
   ctggtttgtg gacaacaaag aagtacacac agcctggacg cagccccgtg aagctcagta    360
   caacagtacc ttccgagtgg tcagtgccct ccccatccag caccaggact ggatgagggg    420
   caaggagttc aaatgcaagg tcaacaacaa agccctccca gcccccatcg agagaaccat    480
   ctcaaaaccc aaaggaagag cccagacacc tcaagtatac accatacccc cacctcgtga    540
   acaaatgtcc aagaagaagg ttagtctgac ctgcctggtc accaacttct tctctgaagc    600
   catcagtgtg gagtgggaaa ggaacggaga actggagcag gattacaaga acactccacc    660
   catcctggac tcggatggga cctacttcct ctacagcaag ctcactgtgg atacagacag    720
   ttggttgcaa ggagaaattt ttacctgctc cgtggtgcat gaggctctcc ataaccacca    780
   cacacagaag aacctgtctc gctcccctgg taaatgagaa cagcatctag cggccgctcg    840
   aggccggcaa ggccggatcc agacatgata agatacattg atgagtttgg acaaaccaca    900
   actagaatgc agtgaaaaaa atgctttatt tgtgaaattt gtgatgctat tgctttattt    960
   gtaaccatta taagctgcaa taaacaagtt aacaacaaca attgcattca ttttatgttt   1020
   caggttcagg gggaggtgtg ggaggttttt taaagcaagt aaaacctcta caaatgtggt   1080
   atggctgatt atgatccggc tgcctcgcgc gtttcggtga tgacggtgaa aacctctgac   1140
   acatgcagct cccggagacg gtcacagctt gtctgtaagc ggatgccggg agcagacaag   1200
   cccgtcaggc gtcagcgggt gttggcgggt gtcggggcgc agccatgagg tcgactctag   1260
   aggatcgatg ccccgccccg gacgaactaa acctgactac gacatctctg ccccttcttc   1320
   gcggggcagt gcatgtaatc ccttcagttg gttggtacaa cttgccaact gggccctgtt   1380
   ccacatgtga cacggggggg gaccaaacac aaaggggttc tctgactgta gttgacatcc   1440
   ttataaatgg atgtgcacat ttgccaacac tgagtggctt tcatcctgga gcagactttg   1500
   cagtctgtgg actgcaacac aacattgcct ttatgtgtaa ctcttggctg aagctcttac   1560
   accaatgctg ggggacatgt acctcccagg ggcccaggaa gactacggga ggctacacca   1620
   acgtcaatca gaggggcctg tgtagctacc gataagcgga ccctcaagag ggcattagca   1680
   atagtgttta taaggccccc ttgttaaccc taaacgggta gcatatgctt cccgggtagt 1740
   agtatatact atccagacta accctaattc aatagcatat gttacccaac gggaagcata 1800
   tgctatcgaa ttagggttag taaaagggtc ctaaggaaca gcgatatctc ccaccccatg 1860
   agctgtcacg gttttattta catggggtca ggattccacg agggtagtga accattttag 1920
   tcacaagggc agtggctgaa gatcaaggag cgggcagtga actctcctga atcttcgcct 1980
   gcttcttcat tctccttcgt ttagctaata gaataactgc tgagttgtga acagtaaggt 2040
   gtatgtgagg tgctcgaaaa caaggtttca ggtgacgccc ccagaataaa atttggacgg 2100
   ggggttcagt ggtggcattg tgctatgaca ccaatataac cctcacaaac cccttgggca 2160
   ataaatacta gtgtaggaat gaaacattct gaatatcttt aacaatagaa atccatgggg 2220
   tggggacaag ccgtaaagac tggatgtcca tctcacacga atttatggct atgggcaaca 2280
   cataatccta gtgcaatatg atactggggt tattaagatg tgtcccaggc agggaccaag 2340
   acaggtgaac catgttgtta cactctattt gtaacaaggg gaaagagagt ggacgccgac 2400
   agcagcggac tccactggtt gtctctaaca cccccgaaaa ttaaacgggg ctccacgcca 2460
   atggggccca taaacaaaga caagtggcca ctcttttttt tgaaattgtg gagtgggggc 2520
   acgcgtcagc ccccacacgc cgccctgcgg ttttggactg taaaataagg gtgtaataac 2580
   ttggctgatt gtaaccccgc taaccactgc ggtcaaacca cttgcccaca aaaccactaa 2640
   tggcaccccg gggaatacct gcataagtag gtgggcgggc caagataggg gcgcgattgc 2700
   tgcgatctgg aggacaaatt acacacactt gcgcctgagc gccaagcaca gggttgttgg 2760
   tcctcatatt cacgaggtcg ctgagagcac ggtgggctaa tgttgccatg ggtagcatat 2820
   actacccaaa tatctggata gcatatgcta tcctaatcta tatctgggta gcataggcta 2880
   tcctaatcta tatctgggta gcatatgcta tcctaatcta tatctgggta gtatatgcta 2940
   tcctaattta tatctgggta gcataggcta tcctaatcta tatctgggta gcatatgcta 3000
   tcctaatcta tatctgggta gtatatgcta tcctaatctg tatccgggta gcatatgcta 3060
   tcctaataga gattagggta gtatatgcta tcctaattta tatctgggta gcatatacta 3120
   cccaaatatc tggatagcat atgctatcct aatctatatc tgggtagcat atgctatcct 3180
   aatctatatc tgggtagcat aggctatcct aatctatatc tgggtagcat atgctatcct 3240
   aatctatatc tgggtagtat atgctatcct aatttatatc tgggtagcat aggctatcct 3300
   aatctatatc tgggtagcat atgctatcct aatctatatc tgggtagtat atgctatcct 3360
   aatctgtatc cgggtagcat atgctatcct catgcatata cagtcagcat atgataccca 3420
   gtagtagagt gggagtgcta tcctttgcat atgccgccac ctcccaaggg ggcgtgaatt 3480
   ttcgctgctt gtccttttcc tgctggttgc tcccattctt aggtgaattt aaggaggcca 3540
   ggctaaagcc gtcgcatgtc tgattgctca ccaggtaaat gtcgctaatg ttttccaacg     3600
   cgagaaggtg ttgagcgcgg agctgagtga cgtgacaaca tgggtatgcc caattgcccc     3660
   atgttgggag gacgaaaatg gtgacaagac agatggccag aaatacacca acagcacgca     3720
   tgatgtctac tggggattta ttctttagtg cgggggaata cacggctttt aatacgattg     3780
   agggcgtctc ctaacaagtt acatcactcc tgcccttcct caccctcatc tccatcacct     3840
   ccttcatctc cgtcatctcc gtcatcaccc tccgcggcag ccccttccac cataggtgga     3900
   aaccagggag gcaaatctac tccatcgtca aagctgcaca cagtcaccct gatattgcag     3960
   gtaggagcgg gctttgtcat aacaaggtcc ttaatcgcat ccttcaaaac ctcagcaaat     4020
   atatgagttt gtaaaaagac catgaaataa cagacaatgg actcccttag cgggccaggt     4080
   tgtgggccgg gtccaggggc cattccaaag gggagacgac tcaatggtgt aagacgacat     4140
   tgtggaatag caagggcagt tcctcgcctt aggttgtaaa gggaggtctt actacctcca     4200
   tatacgaaca caccggcgac ccaagttcct tcgtcggtag tcctttctac gtgactccta     4260
   gccaggagag ctcttaaacc ttctgcaatg ttctcaaatt tcgggttgga acctccttga     4320
   ccacgatgct ttccaaacca ccctcctttt ttgcgcctgc ctccatcacc ctgaccccgg     4380
   ggtccagtgc ttgggccttc tcctgggtca tctgcggggc cctgctctat cgctcccggg     4440
   ggcacgtcag gctcaccatc tgggccacct tcttggtggt attcaaaata atcggcttcc     4500
   cctacagggt ggaaaaatgg ccttctacct ggaggggccc tgcgcggtgg agacccggat     4560
   gatgatgact gactactggg actcctgggc ctcttttctc cacgtccacg acctctcccc     4620
   ctggctcttt cacgacttcc ccccctggct ctttcacgtc ctctaccccg gcggcctcca     4680
   ctacctcctc gaccccggcc tccactacct cctcgacccc ggcctccact gcctcctcga     4740
   ccccggcctc cacctcctgc tcctgcccct cctgctcctg cccctcctcc tgctcctgcc     4800
   cctcctgccc ctcctgctcc tgcccctcct gcccctcctg ctcctgcccc tcctgcccct     4860
   cctgctcctg cccctcctgc ccctcctcct gctcctgccc ctcctgcccc tcctcctgct     4920
   cctgcccctc ctgcccctcc tgctcctgcc cctcctgccc ctcctgctcc tgcccctcct     4980
   gcccctcctg ctcctgcccc tcctgctcct gcccctcctg ctcctgcccc tcctgctcct     5040
   gcccctcctg cccctcctgc ccctcctcct gctcctgccc ctcctgctcc tgcccctcct     5100
   gcccctcctg cccctcctgc tcctgcccct cctcctgctc ctgcccctcc tgcccctcct     5160
   gcccctcctc ctgctcctgc ccctcctgcc cctcctcctg ctcctgcccc tcctcctgct     5220
   cctgcccctc ctgcccctcc tgcccctcct cctgctcctg cccctcctgc ccctcctcct     5280
   gctcctgccc ctcctcctgc tcctgcccct cctgcccctc ctgcccctcc tcctgctcct     5340
   gcccctcctc ctgctcctgc ccctcctgcc cctcctgccc ctcctgcccc tcctcctgct     5400
   cctgcccctc ctcctgctcc tgcccctcct gctcctgccc ctcccgctcc tgctcctgct     5460
   cctgttccac cgtgggtccc tttgcagcca atgcaacttg gacgtttttg gggtctccgg 5520
   acaccatctc tatgtcttgg ccctgatcct gagccgcccg gggctcctgg tcttccgcct 5580
   cctcgtcctc gtcctcttcc ccgtcctcgt ccatggttat caccccctct tctttgaggt 5640
   ccactgccgc cggagccttc tggtccagat gtgtctccct tctctcctag gccatttcca 5700
   ggtcctgtac ctggcccctc gtcagacatg attcacacta aaagagatca atagacatct 5760
   ttattagacg acgctcagtg aatacaggga gtgcagactc ctgccccctc caacagcccc 5820
   cccaccctca tccccttcat ggtcgctgtc agacagatcc aggtctgaaa attccccatc 5880
   ctccgaacca tcctcgtcct catcaccaat tactcgcagc ccggaaaact cccgctgaac 5940
   atcctcaaga tttgcgtcct gagcctcaag ccaggcctca aattcctcgt cccccttttt 6000
   gctggacggt agggatgggg attctcggga cccctcctct tcctcttcaa ggtcaccaga 6060
   cagagatgct actggggcaa cggaagaaaa gctgggtgcg gcctgtgagg atcagcttat 6120
   cgatgataag ctgtcaaaca tgagaattct tgaagacgaa agggcctcgt gatacgccta 6180
   tttttatagg ttaatgtcat gataataatg gtttcttaga cgtcaggtgg cacttttcgg 6240
   ggaaatgtgc gcggaacccc tatttgttta tttttctaaa tacattcaaa tatgtatccg 6300
   ctcatgagac aataaccctg ataaatgctt caataatatt gaaaaaggaa gagtatgagt 6360
   attcaacatt tccgtgtcgc ccttattccc ttttttgcgg cattttgcct tcctgttttt 6420
   gctcacccag aaacgctggt gaaagtaaaa gatgctgaag atcagttggg tgcacgagtg 6480
   ggttacatcg aactggatct caacagcggt aagatccttg agagttttcg ccccgaagaa 6540
   cgttttccaa tgatgagcac ttttaaagtt ctgctatgtg gcgcggtatt atcccgtgtt 6600
   gacgccgggc aagagcaact cggtcgccgc atacactatt ctcagaatga cttggttgag 6660
   tactcaccag tcacagaaaa gcatcttacg gatggcatga cagtaagaga attatgcagt 6720
   gctgccataa ccatgagtga taacactgcg gccaacttac ttctgacaac gatcggagga 6780
   ccgaaggagc taaccgcttt tttgcacaac atgggggatc atgtaactcg ccttgatcgt 6840
   tgggaaccgg agctgaatga agccatacca aacgacgagc gtgacaccac gatgcctgca 6900
   gcaatggcaa caacgttgcg caaactatta actggcgaac tacttactct agcttcccgg 6960
   caacaattaa tagactggat ggaggcggat aaagttgcag gaccacttct gcgctcggcc 7020
   cttccggctg gctggtttat tgctgataaa tctggagccg gtgagcgtgg gtctcgcggt 7080
   atcattgcag cactggggcc agatggtaag ccctcccgta tcgtagttat ctacacgacg 7140
   gggagtcagg caactatgga tgaacgaaat agacagatcg ctgagatagg tgcctcactg 7200
   attaagcatt ggtaactgtc agaccaagtt tactcatata tactttagat tgatttaaaa 7260
   cttcattttt aatttaaaag gatctaggtg aagatccttt ttgataatct catgaccaaa 7320
   atcccttaac gtgagttttc gttccactga gcgtcagacc ccgtagaaaa gatcaaagga 7380
   tcttcttgag atcctttttt tctgcgcgta atctgctgct tgcaaacaaa aaaaccaccg 7440
   ctaccagcgg tggtttgttt gccggatcaa gagctaccaa ctctttttcc gaaggtaact 7500
   ggcttcagca gagcgcagat accaaatact gtccttctag tgtagccgta gttaggccac 7560
   cacttcaaga actctgtagc accgcctaca tacctcgctc tgctaatcct gttaccagtg 7620
   gctgctgcca gtggcgataa gtcgtgtctt accgggttgg actcaagacg atagttaccg 7680
   gataaggcgc agcggtcggg ctgaacgggg ggttcgtgca cacagcccag cttggagcga 7740
   acgacctaca ccgaactgag atacctacag cgtgagctat gagaaagcgc cacgcttccc 7800
   gaagggagaa aggcggacag gtatccggta agcggcaggg tcggaacagg agagcgcacg 7860
   agggagcttc cagggggaaa cgcctggtat ctttatagtc ctgtcgggtt tcgccacctc 7920
   tgacttgagc gtcgattttt gtgatgctcg tcaggggggc ggagcctatg gaaaaacgcc 7980
   agcaacgcgg cctttttacg gttcctggcc ttttgctggc cttgaagctg tccctgatgg 8040
   tcgtcatcta cctgcctgga cagcatggcc tgcaacgcgg gcatcccgat gccgccggaa 8100
   gcgagaagaa tcataatggg gaaggccatc cagcctcgcg tcgcgaacgc cagcaagacg 8160
   tagcccagcg cgtcggcccc gagatgcgcc gcgtgcggct gctggagatg gcggacgcga 8220
   tggatatgtt ctgccaaggg ttggtttgcg cattcacagt tctccgcaag aattgattgg 8280
   ctccaattct tggagtggtg aatccgttag cgaggtgccg ccctgcttca tccccgtggc 8340
   ccgttgctcg cgtttgctgg cggtgtcccc ggaagaaata tatttgcatg tctttagttc 8400
   tatgatgaca caaaccccgc ccagcgtctt gtcattggcg aattcgaaca cgcagatgca 8460
   gtcggggcgg cgcggtccga ggtccacttc gcatattaag gtgacgcgtg tggcctcgaa 8520
   caccgagcga ccctgcagcg acccgcttaa cagcgtcaac agcgtgccgc agatcccggg 8580
   gggcaatgag atatgaaaaa gcctgaactc accgcgacgt ctgtcgagaa gtttctgatc 8640
   gaaaagttcg acagcgtctc cgacctgatg cagctctcgg agggcgaaga atctcgtgct 8700
   ttcagcttcg atgtaggagg gcgtggatat gtcctgcggg taaatagctg cgccgatggt 8760
   ttctacaaag atcgttatgt ttatcggcac tttgcatcgg ccgcgctccc gattccggaa 8820
   gtgcttgaca ttggggaatt cagcgagagc ctgacctatt gcatctcccg ccgtgcacag 8880
   ggtgtcacgt tgcaagacct gcctgaaacc gaactgcccg ctgttctgca gccggtcgcg 8940
   gaggccatgg atgcgatcgc tgcggccgat cttagccaga cgagcgggtt cggcccattc 9000
   ggaccgcaag gaatcggtca atacactaca tggcgtgatt tcatatgcgc gattgctgat 9060
   ccccatgtgt atcactggca aactgtgatg gacgacaccg tcagtgcgtc cgtcgcgcag 9120
   gctctcgatg agctgatgct ttgggccgag gactgccccg aagtccggca cctcgtgcac 9180
   gcggatttcg gctccaacaa tgtcctgacg gacaatggcc gcataacagc ggtcattgac 9240
   tggagcgagg cgatgttcgg ggattcccaa tacgaggtcg ccaacatctt cttctggagg 9300
   ccgtggttgg cttgtatgga gcagcagacg cgctacttcg agcggaggca tccggagctt 9360
   gcaggatcgc cgcggctccg ggcgtatatg ctccgcattg gtcttgacca actctatcag 9420
   agcttggttg acggcaattt cgatgatgca gcttgggcgc agggtcgatg cgacgcaatc 9480
   gtccgatccg gagccgggac tgtcgggcgt acacaaatcg cccgcagaag cgcggccgtc 9540
   tggaccgatg gctgtgtaga agtactcgcc gatagtggaa accgacgccc cagcactcgt 9600
   ccggatcggg agatggggga ggctaactga aacacggaag gagacaatac cggaaggaac 9660
   ccgcgctatg acggcaataa aaagacagaa taaaacgcac gggtgttggg tcgtttgttc 9720
   ataaacgcgg ggttcggtcc cagggctggc actctgtcga taccccaccg agaccccatt 9780
   ggggccaata cgcccgcgtt tcttcctttt ccccacccca ccccccaagt tcgggtgaag 9840
   gcccagggct cgcagccaac gtcggggcgg caggccctgc catagccact ggccccgtgg 9900
   gttagggacg gggtccccca tggggaatgg tttatggttc gtgggggtta ttattttggg 9960
   cgttgcgtgg ggtcaggtcc acgactggac tgagcagaca gacccatggt ttttggatgg 10020
   cctgggcatg gaccgcatgt actggcgcga cacgaacacc gggcgtctgt ggctgccaaa 10080
   cacccccgac ccccaaaaac caccgcgcgg atttctggcg tgccaagcta gtcgaccaat 10140
   tctcatgttt gacagcttat catcgcagat ccgggcaacg ttgttgccat tgctgcaggc 10200
   gcagaactgg taggtatgga agatctatac attgaatcaa tattggcaat tagccatatt 10260
   agtcattggt tatatagcat aaatcaatat tggctattgg ccattgcata cgttgtatct 10320
   atatcataat atgtacattt atattggctc atgtccaata tgaccgccat gttgacattg 10380
   attattgact agttattaat agtaatcaat tacggggtca ttagttcata gcccatatat 10440
   ggagttccgc gttacataac ttacggtaaa tggcccgcct ggctgaccgc ccaacgaccc 10500
   ccgcccattg acgtcaataa tgacgtatgt tcccatagta acgccaatag ggactttcca 10560
   ttgacgtcaa tgggtggagt atttacggta aactgcccac ttggcagtac atcaagtgta 10620
   tcatatgcca agtccgcccc ctattgacgt caatgacggt aaatggcccg cctggcatta 10680
   tgcccagtac atgaccttac gggactttcc tacttggcag tacatctacg tattagtcat 10740
   cgctattacc atggtgatgc ggttttggca gtacaccaat gggcgtggat agcggtttga 10800
   ctcacgggga tttccaagtc tccaccccat tgacgtcaat gggagtttgt tttggcacca 10860
   aaatcaacgg gactttccaa aatgtcgtaa taaccccgcc ccgttgacgc aaatgggcgg 10920
   taggcgtgta cggtgggagg tctatataag cagagctcgt ttagtgaacc gtcagatctc 10980
   tagaagctgg gtac 10994
<210> 9
   <211> 12539
   <212> DNA
   <213> artificial
<220>
   <223> plasmid vector encoding Fc-tag IL-12 fusion construct
<400> 9
   gtacctcgca gcaaagcaag atgtgtcctc agaagctaac catctcctgg tttgccatcg 60
   ttttgctggt gtctccactc atggccatgt gggagctgga gaaagacgtt tatgttgtag 120
   aggtggactg gactcccgat gcccctggag aaacagtgaa cctcacctgt gacacgcctg 180
   aagaagatga catcacctgg acctcagacc agagacatgg agtcataggc tctggaaaga 240
   ccctgaccat cactgtcaaa gagtttctag atgctggcca gtacacctgc cacaaaggag 300
   gcgagactct gagccactca catctgctgc tccacaagaa ggaaaatgga atttggtcca 360
   ctgaaatttt aaaaaatttc aaaaacaaga ctttcctgaa gtgtgaagca ccaaattact 420
   ccggacggtt cacgtgctca tggctggtgc aaagaaacat ggacttgaag ttcaacatca 480
   agagcagtag cagttcccct gactctcggg cagtgacatg tggaatggcg tctctgtctg 540
   cagagaaggt cacactggac caaagggact atgagaagta ttcagtgtcc tgccaggagg 600
   atgtcacctg cccaactgcc gaggagaccc tgcccattga actggcgttg gaagcacggc 660
   agcagaataa atatgagaac tacagcacca gcttcttcat cagggacatc atcaaaccag 720
   acccgcccaa gaacttgcag atgaagcctt tgaagaactc acaggtggag gtcagctggg 780
   agtaccctga ctcctggagc actccccatt cctacttctc cctcaagttc tttgttcgaa 840
   tccagcgcaa gaaagaaaag atgaaggaga cagaggaggg gtgtaaccag aaaggtgcgt 900
   tcctcgtaga gaagacatct accgaagtcc aatgcaaagg cgggaatgtc tgcgtgcaag 960
   ctcaggatcg ctattacaat tcctcgtgca gcaagtgggc atgtgttccc tgcagggtcc 1020
   gatccggagg cggtggctcg ggcggtggtg ggtcgggtgg cggcggatcc agggtcattc 1080
   cagtctctgg acctgccagg tgtcttagcc agtcccgaaa cctgctgaag accacagatg 1140
   acatggtgaa gacggccaga gaaaaactga aacattattc ctgcactgct gaagacatcg 1200
   atcatgaaga catcacacgg gaccaaacca gcacattgaa gacctgttta ccactggaac 1260
   tacacaagaa cgagagttgc ctggctacta gagagacttc ttccacaaca agagggagct 1320
   gcctgccccc acagaagacg tctttgatga tgaccctgtg ccttggtagc atctatgagg 1380
   acttgaagat gtaccagaca gagttccagg ccatcaacgc agcacttcag aatcacaacc 1440
   atcagcagat cattctagac aagggcatgc tggtggccat cgatgagctg atgcagtctc 1500
   tgaatcataa tggcgagact ctgcgccaga aacctcctgt gggagaagca gacccttaca 1560
   gagtgaaaat gaagctctgc atcctgcttc acgccttcag cacccgcgtc gtgaccatca 1620
   acagggtgat gggctatctg agctccgcct tgatcaagag aatcgagcct agaataccca 1680
   agcccagtac ccccccaggt tcttcatgcc cacctggtaa catcttgggt ggaccatccg 1740
   tcttcatctt ccccccaaag cccaaggatg cactcatgat ctccctaacc cccaaggtta 1800
   cgtgtgtggt ggtggatgtg agcgaggatg acccagatgt ccatgtcagc tggtttgtgg 1860
   acaacaaaga agtacacaca gcctggacgc agccccgtga agctcagtac aacagtacct 1920
   tccgagtggt cagtgccctc cccatccagc accaggactg gatgaggggc aaggagttca 1980
   aatgcaaggt caacaacaaa gccctcccag cccccatcga gagaaccatc tcaaaaccca 2040
   aaggaagagc ccagacacct caagtataca ccataccccc acctcgtgaa caaatgtcca 2100
   agaagaaggt tagtctgacc tgcctggtca ccaacttctt ctctgaagcc atcagtgtgg 2160
   agtgggaaag gaacggagaa ctggagcagg attacaagaa cactccaccc atcctggact 2220
   cggatgggac ctacttcctc tacagcaagc tcactgtgga tacagacagt tggttgcaag 2280
   gagaaatttt tacctgctcc gtggtgcatg aggctctcca taaccaccac acacagaaga 2340
   acctgtctcg ctcccctggt aaatgagaac agcatctagc ggccgctcga ggccggcaag 2400
   gccggatcca gacatgataa gatacattga tgagtttgga caaaccacaa ctagaatgca 2460
   gtgaaaaaaa tgctttattt gtgaaatttg tgatgctatt gctttatttg taaccattat 2520
   aagctgcaat aaacaagtta acaacaacaa ttgcattcat tttatgtttc aggttcaggg 2580
   ggaggtgtgg gaggtttttt aaagcaagta aaacctctac aaatgtggta tggctgatta 2640
   tgatccggct gcctcgcgcg tttcggtgat gacggtgaaa acctctgaca catgcagctc 2700
   ccggagacgg tcacagcttg tctgtaagcg gatgccggga gcagacaagc ccgtcaggcg 2760
   tcagcgggtg ttggcgggtg tcggggcgca gccatgaggt cgactctaga ggatcgatgc 2820
   cccgccccgg acgaactaaa cctgactacg acatctctgc cccttcttcg cggggcagtg 2880
   catgtaatcc cttcagttgg ttggtacaac ttgccaactg ggccctgttc cacatgtgac 2940
   acgggggggg accaaacaca aaggggttct ctgactgtag ttgacatcct tataaatgga 3000
   tgtgcacatt tgccaacact gagtggcttt catcctggag cagactttgc agtctgtgga 3060
   ctgcaacaca acattgcctt tatgtgtaac tcttggctga agctcttaca ccaatgctgg 3120
   gggacatgta cctcccaggg gcccaggaag actacgggag gctacaccaa cgtcaatcag 3180
   aggggcctgt gtagctaccg ataagcggac cctcaagagg gcattagcaa tagtgtttat 3240
   aaggccccct tgttaaccct aaacgggtag catatgcttc ccgggtagta gtatatacta 3300
   tccagactaa ccctaattca atagcatatg ttacccaacg ggaagcatat gctatcgaat 3360
   tagggttagt aaaagggtcc taaggaacag cgatatctcc caccccatga gctgtcacgg 3420
   ttttatttac atggggtcag gattccacga gggtagtgaa ccattttagt cacaagggca 3480
   gtggctgaag atcaaggagc gggcagtgaa ctctcctgaa tcttcgcctg cttcttcatt 3540
   ctccttcgtt tagctaatag aataactgct gagttgtgaa cagtaaggtg tatgtgaggt 3600
   gctcgaaaac aaggtttcag gtgacgcccc cagaataaaa tttggacggg gggttcagtg 3660
   gtggcattgt gctatgacac caatataacc ctcacaaacc ccttgggcaa taaatactag 3720
   tgtaggaatg aaacattctg aatatcttta acaatagaaa tccatggggt ggggacaagc 3780
   cgtaaagact ggatgtccat ctcacacgaa tttatggcta tgggcaacac ataatcctag 3840
   tgcaatatga tactggggtt attaagatgt gtcccaggca gggaccaaga caggtgaacc 3900
   atgttgttac actctatttg taacaagggg aaagagagtg gacgccgaca gcagcggact 3960
   ccactggttg tctctaacac ccccgaaaat taaacggggc tccacgccaa tggggcccat 4020
   aaacaaagac aagtggccac tctttttttt gaaattgtgg agtgggggca cgcgtcagcc 4080
   cccacacgcc gccctgcggt tttggactgt aaaataaggg tgtaataact tggctgattg 4140
   taaccccgct aaccactgcg gtcaaaccac ttgcccacaa aaccactaat ggcaccccgg 4200
   ggaatacctg cataagtagg tgggcgggcc aagatagggg cgcgattgct gcgatctgga 4260
   ggacaaatta cacacacttg cgcctgagcg ccaagcacag ggttgttggt cctcatattc 4320
   acgaggtcgc tgagagcacg gtgggctaat gttgccatgg gtagcatata ctacccaaat 4380
   atctggatag catatgctat cctaatctat atctgggtag cataggctat cctaatctat 4440
   atctgggtag catatgctat cctaatctat atctgggtag tatatgctat cctaatttat 4500
   atctgggtag cataggctat cctaatctat atctgggtag catatgctat cctaatctat 4560
   atctgggtag tatatgctat cctaatctgt atccgggtag catatgctat cctaatagag 4620
   attagggtag tatatgctat cctaatttat atctgggtag catatactac ccaaatatct 4680
   ggatagcata tgctatccta atctatatct gggtagcata tgctatccta atctatatct 4740
   gggtagcata ggctatccta atctatatct gggtagcata tgctatccta atctatatct 4800
   gggtagtata tgctatccta atttatatct gggtagcata ggctatccta atctatatct 4860
   gggtagcata tgctatccta atctatatct gggtagtata tgctatccta atctgtatcc 4920
   gggtagcata tgctatcctc atgcatatac agtcagcata tgatacccag tagtagagtg 4980
   ggagtgctat cctttgcata tgccgccacc tcccaagggg gcgtgaattt tcgctgcttg 5040
   tccttttcct gctggttgct cccattctta ggtgaattta aggaggccag gctaaagccg 5100
   tcgcatgtct gattgctcac caggtaaatg tcgctaatgt tttccaacgc gagaaggtgt 5160
   tgagcgcgga gctgagtgac gtgacaacat gggtatgccc aattgcccca tgttgggagg 5220
   acgaaaatgg tgacaagaca gatggccaga aatacaccaa cagcacgcat gatgtctact 5280
   ggggatttat tctttagtgc gggggaatac acggctttta atacgattga gggcgtctcc 5340
   taacaagtta catcactcct gcccttcctc accctcatct ccatcacctc cttcatctcc 5400
   gtcatctccg tcatcaccct ccgcggcagc cccttccacc ataggtggaa accagggagg 5460
   caaatctact ccatcgtcaa agctgcacac agtcaccctg atattgcagg taggagcggg 5520
   ctttgtcata acaaggtcct taatcgcatc cttcaaaacc tcagcaaata tatgagtttg 5580
   taaaaagacc atgaaataac agacaatgga ctcccttagc gggccaggtt gtgggccggg 5640
   tccaggggcc attccaaagg ggagacgact caatggtgta agacgacatt gtggaatagc 5700
   aagggcagtt cctcgcctta ggttgtaaag ggaggtctta ctacctccat atacgaacac 5760
   accggcgacc caagttcctt cgtcggtagt cctttctacg tgactcctag ccaggagagc 5820
   tcttaaacct tctgcaatgt tctcaaattt cgggttggaa cctccttgac cacgatgctt 5880
   tccaaaccac cctccttttt tgcgcctgcc tccatcaccc tgaccccggg gtccagtgct 5940
   tgggccttct cctgggtcat ctgcggggcc ctgctctatc gctcccgggg gcacgtcagg 6000
   ctcaccatct gggccacctt cttggtggta ttcaaaataa tcggcttccc ctacagggtg 6060
   gaaaaatggc cttctacctg gagggggcct gcgcggtgga gacccggatg atgatgactg 6120
   actactggga ctcctgggcc tcttttctcc acgtccacga cctctccccc tggctctttc 6180
   acgacttccc cccctggctc tttcacgtcc tctaccccgg cggcctccac tacctcctcg 6240
   accccggcct ccactacctc ctcgaccccg gcctccactg cctcctcgac cccggcctcc 6300
   acctcctgct cctgcccctc ctgctcctgc ccctcctcct gctcctgccc ctcctgcccc 6360
   tcctgctcct gcccctcctg cccctcctgc tcctgcccct cctgcccctc ctgctcctgc 6420
   ccctcctgcc cctcctcctg ctcctgcccc tcctgcccct cctcctgctc ctgcccctcc 6480
   tgcccctcct gctcctgccc ctcctgcccc tcctgctcct gcccctcctg cccctcctgc 6540
   tcctgcccct cctgctcctg cccctcctgc tcctgcccct cctgctcctg cccctcctgc 6600
   ccctcctgcc cctcctcctg ctcctgcccc tcctgctcct gcccctcctg cccctcctgc 6660
   ccctcctgct cctgcccctc ctcctgctcc tgcccctcct gcccctcctg cccctcctcc 6720
   tgctcctgcc cctcctgccc ctcctcctgc tcctgcccct cctcctgctc ctgcccctcc 6780
   tgcccctcct gcccctcctc ctgctcctgc ccctcctgcc cctcctcctg ctcctgcccc 6840
   tcctcctgct cctgcccctc ctgcccctcc tgcccctcct cctgctcctg cccctcctcc 6900
   tgctcctgcc cctcctgccc ctcctgcccc tcctgcccct cctcctgctc ctgcccctcc 6960
   tcctgctcct gcccctcctg ctcctgcccc tcccgctcct gctcctgctc ctgttccacc 7020
   gtgggtccct ttgcagccaa tgcaacttgg acgtttttgg ggtctccgga caccatctct 7080
   atgtcttggc cctgatcctg agccgcccgg ggctcctggt cttccgcctc ctcgtcctcg 7140
   tcctcttccc cgtcctcgtc catggttatc accccctctt ctttgaggtc cactgccgcc 7200
   ggagccttct ggtccagatg tgtctccctt ctctcctagg ccatttccag gtcctgtacc 7260
   tggcccctcg tcagacatga ttcacactaa aagagatcaa tagacatctt tattagacga 7320
   cgctcagtga atacagggag tgcagactcc tgccccctcc aacagccccc ccaccctcat     7380
   ccccttcatg gtcgctgtca gacagatcca ggtctgaaaa ttccccatcc tccgaaccat     7440
   cctcgtcctc atcaccaatt actcgcagcc cggaaaactc ccgctgaaca tcctcaagat     7500
   ttgcgtcctg agcctcaagc caggcctcaa attcctcgtc cccctttttg ctggacggta     7560
   gggatgggga ttctcgggac ccctcctctt cctcttcaag gtcaccagac agagatgcta     7620
   ctggggcaac ggaagaaaag ctgggtgcgg cctgtgagga tcagcttatc gatgataagc     7680
   tgtcaaacat gagaattctt gaagacgaaa gggcctcgtg atacgcctat ttttataggt     7740
   taatgtcatg ataataatgg tttcttagac gtcaggtggc acttttcggg gaaatgtgcg     7800
   cggaacccct atttgtttat ttttctaaat acattcaaat atgtatccgc tcatgagaca     7860
   ataaccctga taaatgcttc aataatattg aaaaaggaag agtatgagta ttcaacattt     7920
   ccgtgtcgcc cttattccct tttttgcggc attttgcctt cctgtttttg ctcacccaga     7980
   aacgctggtg aaagtaaaag atgctgaaga tcagttgggt gcacgagtgg gttacatcga     8040
   actggatctc aacagcggta agatccttga gagttttcgc cccgaagaac gttttccaat     8100
   gatgagcact tttaaagttc tgctatgtgg cgcggtatta tcccgtgttg acgccgggca     8160
   agagcaactc ggtcgccgca tacactattc tcagaatgac ttggttgagt actcaccagt     8220
   cacagaaaag catcttacgg atggcatgac agtaagagaa ttatgcagtg ctgccataac     8280
   catgagtgat aacactgcgg ccaacttact tctgacaacg atcggaggac cgaaggagct     8340
   aaccgctttt ttgcacaaca tgggggatca tgtaactcgc cttgatcgtt gggaaccgga     8400
   gctgaatgaa gccataccaa acgacgagcg tgacaccacg atgcctgcag caatggcaac     8460
   aacgttgcgc aaactattaa ctggcgaact acttactcta gcttcccggc aacaattaat     8520
   agactggatg gaggcggata aagttgcagg accacttctg cgctcggccc ttccggctgg     8580
   ctggtttatt gctgataaat ctggagccgg tgagcgtggg tctcgcggta tcattgcagc     8640
   actggggcca gatggtaagc cctcccgtat cgtagttatc tacacgacgg ggagtcaggc     8700
   aactatggat gaacgaaata gacagatcgc tgagataggt gcctcactga ttaagcattg     8760
   gtaactgtca gaccaagttt actcatatat actttagatt gatttaaaac ttcattttta     8820
   atttaaaagg atctaggtga agatcctttt tgataatctc atgaccaaaa tcccttaacg     8880
   tgagttttcg ttccactgag cgtcagaccc cgtagaaaag atcaaaggat cttcttgaga     8940
   tccttttttt ctgcgcgtaa tctgctgctt gcaaacaaaa aaaccaccgc taccagcggt     9000
   ggtttgtttg ccggatcaag agctaccaac tctttttccg aaggtaactg gcttcagcag     9060
   agcgcagata ccaaatactg tccttctagt gtagccgtag ttaggccacc acttcaagaa     9120
   ctctgtagca ccgcctacat acctcgctct gctaatcctg ttaccagtgg ctgctgccag     9180
   tggcgataag tcgtgtctta ccgggttgga ctcaagacga tagttaccgg ataaggcgca     9240
   gcggtcgggc tgaacggggg gttcgtgcac acagcccagc ttggagcgaa cgacctacac 9300
   cgaactgaga tacctacagc gtgagctatg agaaagcgcc acgcttcccg aagggagaaa 9360
   ggcggacagg tatccggtaa gcggcagggt cggaacagga gagcgcacga gggagcttcc 9420
   agggggaaac gcctggtatc tttatagtcc tgtcgggttt cgccacctct gacttgagcg 9480
   tcgatttttg tgatgctcgt caggggggcg gagcctatgg aaaaacgcca gcaacgcggc 9540
   ctttttacgg ttcctggcct tttgctggcc ttgaagctgt ccctgatggt cgtcatctac 9600
   ctgcctggac agcatggcct gcaacgcggg catcccgatg ccgccggaag cgagaagaat 9660
   cataatgggg aaggccatcc agcctcgcgt cgcgaacgcc agcaagacgt agcccagcgc 9720
   gtcggccccg agatgcgccg cgtgcggctg ctggagatgg cggacgcgat ggatatgttc 9780
   tgccaagggt tggtttgcgc attcacagtt ctccgcaaga attgattggc tccaattctt 9840
   ggagtggtga atccgttagc gaggtgccgc cctgcttcat ccccgtggcc cgttgctcgc 9900
   gtttgctggc ggtgtccccg gaagaaatat atttgcatgt ctttagttct atgatgacac 9960
   aaaccccgcc cagcgtcttg tcattggcga attcgaacac gcagatgcag tcggggcggc 10020
   gcggtccgag gtccacttcg catattaagg tgacgcgtgt ggcctcgaac accgagcgac 10080
   cctgcagcga cccgcttaac agcgtcaaca gcgtgccgca gatcccgggg ggcaatgaga 10140
   tatgaaaaag cctgaactca ccgcgacgtc tgtcgagaag tttctgatcg aaaagttcga 10200
   cagcgtctcc gacctgatgc agctctcgga gggcgaagaa tctcgtgctt tcagcttcga 10260
   tgtaggaggg cgtggatatg tcctgcgggt aaatagctgc gccgatggtt tctacaaaga 10320
   tcgttatgtt tatcggcact ttgcatcggc cgcgctcccg attccggaag tgcttgacat 10380
   tggggaattc agcgagagcc tgacctattg catctcccgc cgtgcacagg gtgtcacgtt 10440
   gcaagacctg cctgaaaccg aactgcccgc tgttctgcag ccggtcgcgg aggccatgga 10500
   tgcgatcgct gcggccgatc ttagccagac gagcgggttc ggcccattcg gaccgcaagg 10560
   aatcggtcaa tacactacat ggcgtgattt catatgcgcg attgctgatc cccatgtgta 10620
   tcactggcaa actgtgatgg acgacaccgt cagtgcgtcc gtcgcgcagg ctctcgatga 10680
   gctgatgctt tgggccgagg actgccccga agtccggcac ctcgtgcacg cggatttcgg 10740
   ctccaacaat gtcctgacgg acaatggccg cataacagcg gtcattgact ggagcgaggc 10800
   gatgttcggg gattcccaat acgaggtcgc caacatcttc ttctggaggc cgtggttggc 10860
   ttgtatggag cagcagacgc gctacttcga gcggaggcat ccggagcttg caggatcgcc 10920
   gcggctccgg gcgtatatgc tccgcattgg tcttgaccaa ctctatcaga gcttggttga 10980
   cggcaatttc gatgatgcag cttgggcgca gggtcgatgc gacgcaatcg tccgatccgg 11040
   agccgggact gtcgggcgta cacaaatcgc ccgcagaagc gcggccgtct ggaccgatgg 11100
   ctgtgtagaa gtactcgccg atagtggaaa ccgacgcccc agcactcgtc cggatcggga    11160
   gatgggggag gctaactgaa acacggaagg agacaatacc ggaaggaacc cgcgctatga    11220
   cggcaataaa aagacagaat aaaacgcacg ggtgttgggt cgtttgttca taaacgcggg    11280
   gttcggtccc agggctggca ctctgtcgat accccaccga gaccccattg gggccaatac    11340
   gcccgcgttt cttccttttc cccaccccac cccccaagtt cgggtgaagg cccagggctc    11400
   gcagccaacg tcggggcggc aggccctgcc atagccactg gccccgtggg ttagggacgg    11460
   ggtcccccat ggggaatggt ttatggttcg tgggggttat tattttgggc gttgcgtggg    11520
   gtcaggtcca cgactggact gagcagacag acccatggtt tttggatggc ctgggcatgg    11580
   accgcatgta ctggcgcgac acgaacaccg ggcgtctgtg gctgccaaac acccccgacc    11640
   cccaaaaacc accgcgcgga tttctggcgt gccaagctag tcgaccaatt ctcatgtttg    11700
   acagcttatc atcgcagatc cgggcaacgt tgttgccatt gctgcaggcg cagaactggt    11760
   aggtatggaa gatctataca ttgaatcaat attggcaatt agccatatta gtcattggtt    11820
   atatagcata aatcaatatt ggctattggc cattgcatac gttgtatcta tatcataata    11880
   tgtacattta tattggctca tgtccaatat gaccgccatg ttgacattga ttattgacta    11940
   gttattaata gtaatcaatt acggggtcat tagttcatag cccatatatg gagttccgcg    12000
   ttacataact tacggtaaat ggcccgcctg gctgaccgcc caacgacccc cgcccattga    12060
   cgtcaataat gacgtatgtt cccatagtaa cgccaatagg gactttccat tgacgtcaat    12120
   gggtggagta tttacggtaa actgcccact tggcagtaca tcaagtgtat catatgccaa    12180
   gtccgccccc tattgacgtc aatgacggta aatggcccgc ctggcattat gcccagtaca    12240
   tgaccttacg ggactttcct acttggcagt acatctacgt attagtcatc gctattacca    12300
   tggtgatgcg gttttggcag tacaccaatg ggcgtggata gcggtttgac tcacggggat    12360
   ttccaagtct ccaccccatt gacgtcaatg ggagtttgtt ttggcaccaa aatcaacggg    12420
   actttccaaa atgtcgtaat aaccccgccc cgttgacgca aatgggcggt aggcgtgtac    12480
   ggtgggaggt ctatataagc agagctcgtt tagtgaaccg tcagatctct agaagctgg     12539